# EUROPÄISCHE PATENTSCHRIFT

(11) **EP 3 158 597 B1**
(45) Veröffentlichungstag und Bekanntmachung des Hinweises auf die Patenterteilung: **23.09.2020**
(21) Anmeldenummer: 15725232.1
(22) Anmeldetag: 22.05.2015
(51) Int. Cl.: H01L 51/30, C07D 209/80, C07D 209/96, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATIÈRES POUR DISPOSITIFS ÉLECTROLUMINESCENTS ORGANIQUES

(30) Priorität: 18.06.2014 EP 14002104
(43) Veröffentlichungstag der Anmeldung: 26.04.2017
(73) Patentinhaber: Merck Patent GmbH, 64293 Darmstadt (DE)
(72) Erfinder: JOOSTEN, Dominik, 60487 Frankfurt am Main (DE); HAYER, Anna, 64293 Darmstadt (DE); MAIER-FLAIG, Florian, 69469 Weinheim (DE); LINGE, Rouven, 64291 Darmstadt (DE); HEIL, Holger, 60389 Frankfurt am Main (DE)
(86) Internationale Anmeldenummer: PCT/EP2015/001059
(87) Internationale Veröffentlichungsnummer: WO 2015/192939

(56) Entgegenhaltungen:
- WO-A1-2013/100467
- US-A1- 2013 256 645
- SUMANT KOWSHIK ET AL: "Ensuring code safety without runtime checks for real-time control systems", PROCEEDINGS OF THE 2002 INTERNATIONAL CONFERENCE ON COMPILERS, ARCHITECTURE, AND SYNTHESIS FOR EMBEDDED SYSTEMS, 1. Oktober 2002 (2002-10-01), Seiten 288-297, XP55198469, DOI: 10.1145/581630.581678 ISBN: 978-1-58-113575-6

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien und deren Ladungstransporteigenschaften können somit auch zu deutlichen Verbesserungen der OLED-Eigenschaften führen.

Gemäß dem Stand der Technik werden unter anderem Indolocarbazolderivate, z. B. gemäß WO 2007/063754 oder WO 2008/056746, Indenocarbazolderivate, z. B. gemäß WO 2010/136109, oder Fluoren- bzw. Spirobifluorenderivate, z. B. gemäß WO 2012/074210, als Matrixmaterialien für phosphoreszierende Emitter in organischen Elektrolumineszenzvorrichtungen eingesetzt. Hier sind weitere Verbesserungen wünschenswert, insbesondere in Bezug auf die Effizienz, die Lebensdauer und die Filmbildung der Materialien.

Aufgabe der vorliegenden Erfindung ist die Bereitstellung von Verbindungen, welche sich für den Einsatz in einer OLED eignen, insbesondere als Matrix-material für phosphoreszierende Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer und/oder die Effizienz. Darüber hinaus weisen diese Verbindungen bei Prozessierung aus Lösung verbesserte Filmbildungseigenschaften auf, da sie gleichzeitig eine hohe Glasübergangstemperatur und eine hohe Löslichkeit aufweisen, was die Prozessierung aus Lösung und das anschließende Ausheizen ermöglicht. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus WO 2013/100467 sind organische Elektrolumineszenzvorrichtungen bekannt, welche heteroaromatische Spiroverbindungen, die mit einer Diarylaminogruppe substituiert sind, als Hostmaterialien enthalten.

Aus US 2013/256645 sind Fluorenderivate für die Verwendung in organischen Elektrolumineszenzvorrichtungen bekannt, welche am Fluorengrundkörper mit einer elektronentransportierenden Gruppe und in 9-Position mit einer lochtransportierenden Gruppe substituiert sind. Dabei kann das Fluoren auch ein Spirobifluoren sein.

Gegenstand der vorliegenden Erfindung ist eine Verbindung gemäß Formel (1) oder Formel (2), 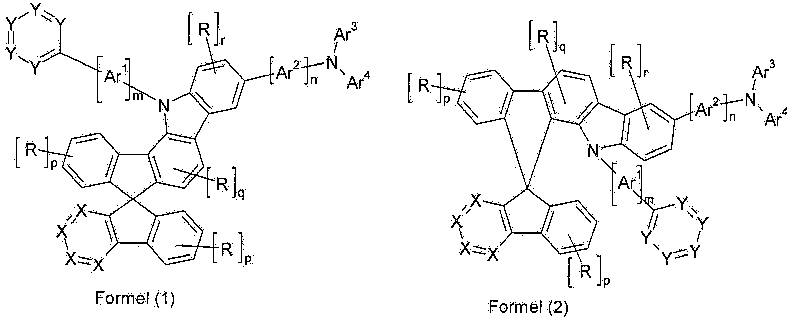 wobei für die verwendeten Symbole und Indizes gilt:
- Y: ist bei jedem Auftreten gleich oder verschieden CR oder N, mit der Maßgabe, dass mindestens eine Gruppe Y für N steht;
- X: ist bei jedem Auftreten gleich oder verschieden CR oder N, wobei maximal zwei Gruppen X für N stehen; oder zwei benachbarte X stehen für eine Gruppe der folgenden Formel (3) oder (4) und die weiteren X stehen gleich oder verschieden für CR oder N, 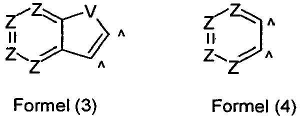 wobei ^ die entsprechenden benachbarten Gruppen X in Formel (1) bzw. Formel (2) andeutet;
- V: ist bei jedem Auftreten gleich oder verschieden NR, C(R)₂, O, S, BR, Si(R)₂ oder C=O;
- Z: ist bei jedem Auftreten gleich oder verschieden CR oder N, wobei maximal zwei Gruppen Z für N stehen;
- Ar¹: ist ein aromatisches oder heteroaromatisches Ringsystem mit 5 bis 40 aromatischen Ringatomen, das mit einem oder mehreren Resten R substituiert sein kann;
- Ar², Ar³, Ar⁴: ist bei jedem Auftreten gleich oder verschieden ein aromatisches oder heteroaromatisches Ringsystem mit 5 bis 40 aromatischen Ringatomen, das mit einem oder mehreren Resten R substituiert sein kann; dabei können Ar² und Ar³ miteinander und/oder Ar³ und Ar⁴ miteinander auch durch eine Einfachbindung oder durch eine Gruppe ausgewählt aus C(R¹)₂, C(R¹)₂-C(R¹)₂, NR1, O oder S verbunden sein;
- R: ist bei jedem Auftreten gleich oder verschieden ausgewählt aus der Gruppe bestehend aus H, D, F, Cl, Br, I, CN, NO₂, N(Ar⁵)₂, N(R¹)₂, C(=O)Ar⁵, C(=O)R¹, P(=O)(Ar⁵)₂, P(Ar⁵)₂, B(Ar⁵)₂, Si(Ar⁵)₃, Si(R¹)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann; dabei können optional zwei benachbarte Substituenten R ein monocyclisches oder polycyclisches, aliphatisches, aromatisches oder heteroaromatisches Ringsystem bilden, das mit einem oder mehreren Resten R¹ substituiert sein kann;
- Ar⁵: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können zwei Reste Ar⁵, welche an dasselbe N-, P-, B- oder Si-Atom binden, auch durch eine Einfachbindung oder eine Brücke, ausgewählt aus N(R¹), C(R¹)₂, C(R¹)₂-C(R¹)₂, O oder S, miteinander verbrückt sein;
- R¹: ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können, wobei zwei oder mehr benachbarte Substituenten R¹ miteinander ein mono- oder polycyclisches, aliphatisches Ringsystem bilden können;
- m: ist 0 oder 1;
- n: ist 0 oder 1;
- p: ist bei jedem Auftreten gleich oder verschieden 0, 1, 2, 3 oder 4;
- q: ist 0, 1 oder 2;
- r: ist 0, 1, 2 oder 3;
dabei ist die folgende Verbindung von der Erfindung ausgenommen:

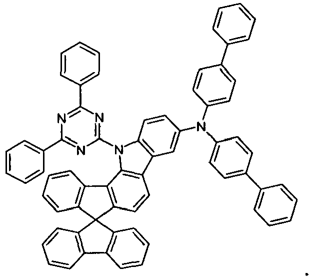

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei enthält die Heteroarylgruppe bevorzugt maximal drei Heteroatome, von denen maximal eines ausgewählt ist aus O oder S und die weiteren Heteroatome N sind. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl oder Bipyridin, werden dagegen nicht als Aryl- oder Heteroarylgruppe, sondern als aromatisches bzw. heteroaromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Dabei enthält das heteroaromatische Ringsystem bevorzugt pro Heteroarylgruppe, die in dem Ringsystem enthalten ist, maximal drei Heteroatome, von denen maximal eines ausgewählt ist aus O oder S und die weiteren Heteroatome N sind. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind, oder Substituenten, die an dasselbe C-, Si-, N-, P- oder B-Atom gebunden sind.

In einer bevorzugten Ausführungsform der Erfindung steht X gleich oder verschieden bei jedem Auftreten für CR oder N, wobei maximal eine Gruppe X pro Cyclus für N steht; oder zwei benachbarte Gruppen X stehen für eine Gruppe der Formel (3), wobei Z gleich oder verschieden bei jedem Auftreten für CR steht und V für NR, C(R)₂, O oder S, bevorzugt für NR oder C(R)₂, steht, und die verbleibenden X stehen für CR. Besonders bevorzugt steht X gleich oder verschieden bei jedem Auftreten für CR.

Bevorzugte Ausführungsformen der Verbindungen gemäß Formel (1) sind die Verbindungen der folgenden Formeln (5), (6) und (7), und bevorzugte Ausführungsformen der Verbindungen der Formel (2) sind die Verbindungen der folgenden Formeln (8), (9) und (10), 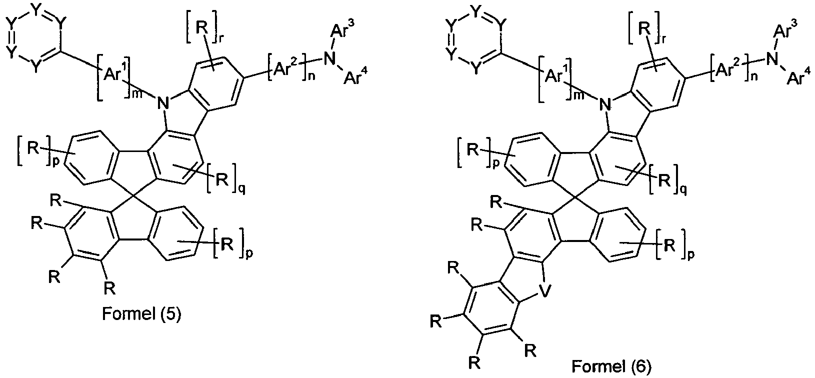 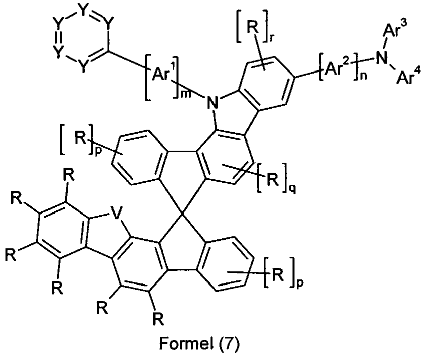 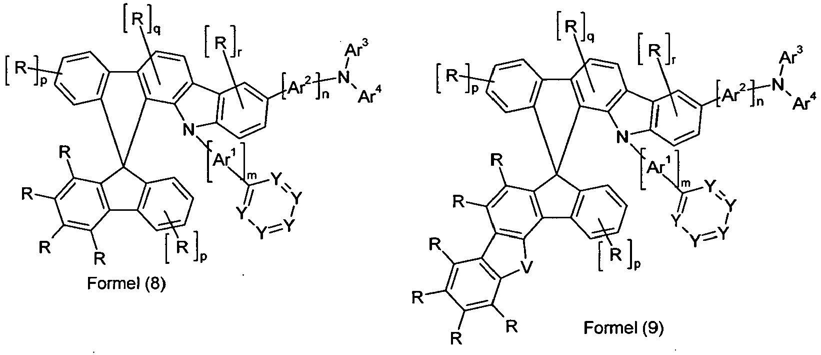 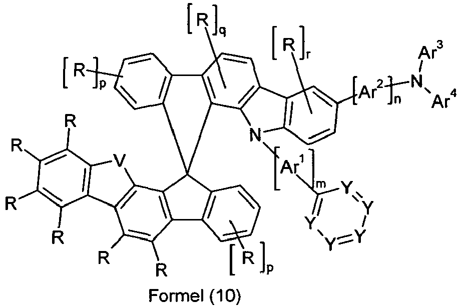 wobei die verwendeten Symbole und Indizes die oben genannten Bedeutungen aufweisen. Dabei steht V bevorzugt für NR, C(R)₂, O oder S, besonders bevorzugt für NR. Es kann bevorzugt sein, wenn für V = C(R)₂ die beiden Reste R miteinander einen Ring bilden und so ein Spirosystem aufspannen.

In einer bevorzugten Ausführungsform der Erfindung ist p bei jedem Auftreten gleich oder verschieden 0, 1 oder 2, besonders bevorzugt 0 oder 1 und ganz besonders bevorzugt gleich 0.

Weiterhin bevorzugt ist q gleich 0 oder 1, besonders bevorzugt gleich 0.

Weiterhin bevorzugt ist r gleich 0 oder 1, besonders bevorzugt gleich 0.

Bevorzugte Ausführungsformen der Strukturen gemäß Formel (5) bis (10) sind die Strukturen der Formeln (5a) bis (10a), 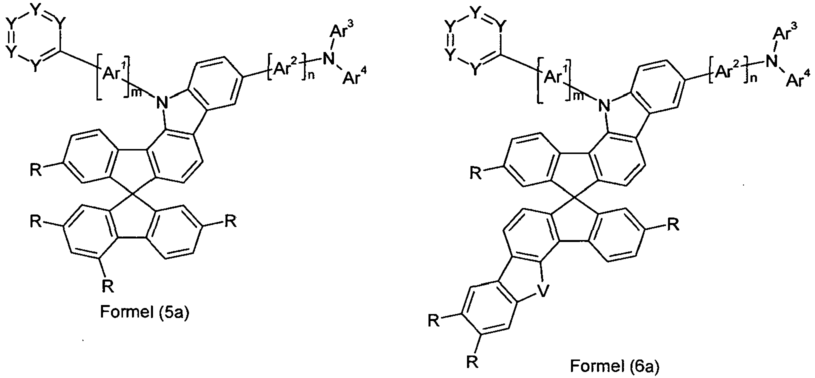 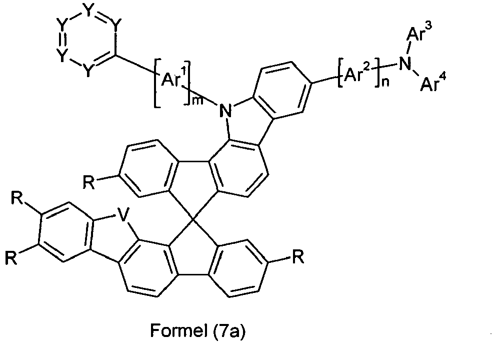 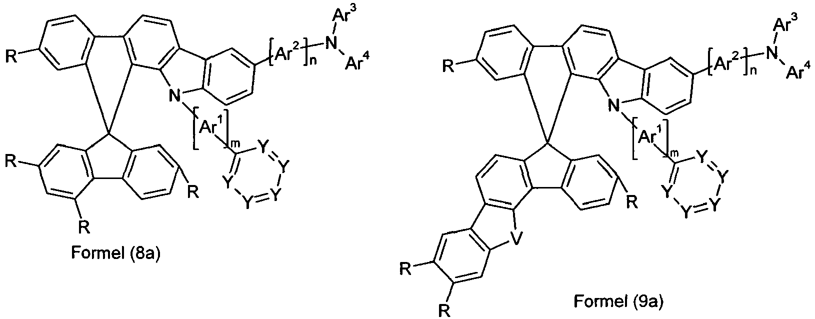 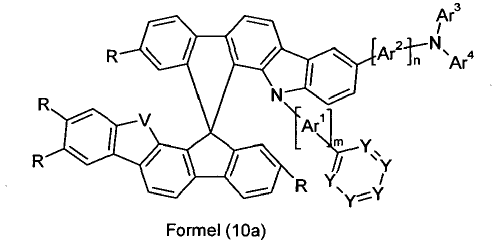 wobei die verwendeten Symbole und Indizes die oben genannten Bedeutungen aufweisen.

Besonders bevorzugte Ausführungsformen der Strukturen gemäß den Formeln (5) bis (10) sind die Strukturen der Formeln (5b) bis (10b), 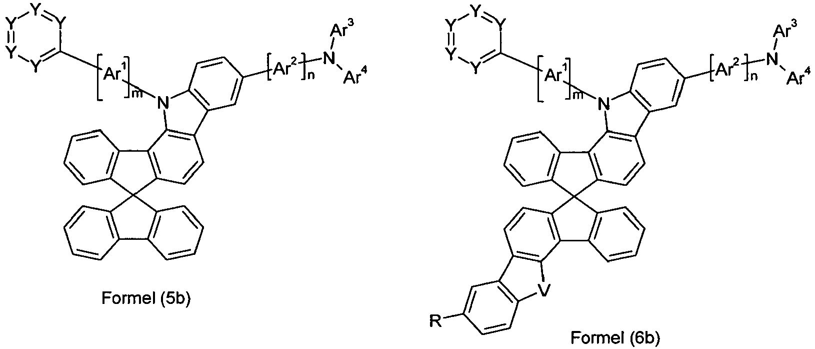 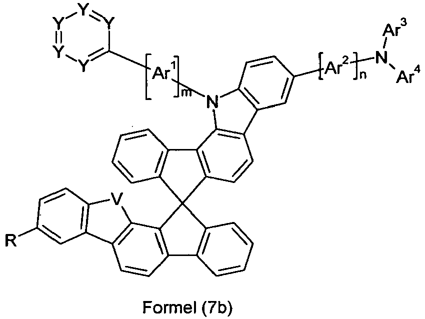 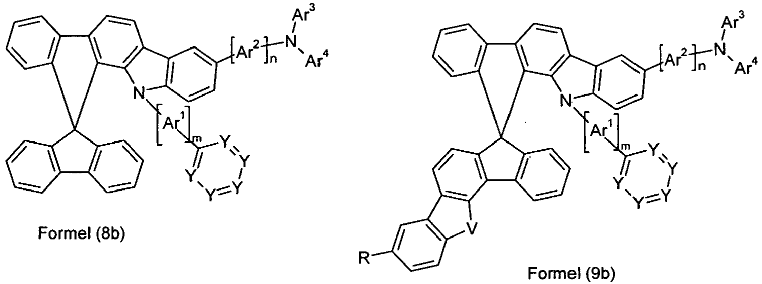 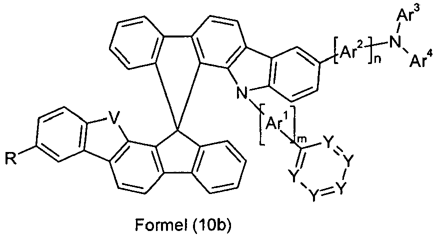 wobei die verwendeten Symbole und Indizes die oben genannten Bedeutungen aufweisen.

In einer weiteren bevorzugten Ausführungsform der Erfindung ist R gleich oder verschieden bei jedem Auftreten ausgewählt aus der Gruppe bestehend aus H, D, F, CN, N(Ar⁵)₂, C(=O)Ar⁵, P(=O)(Ar⁵)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R¹ substituiert sein kann. Besonders bevorzugt ist R gleich oder verschieden bei jedem Auftreten ausgewählt aus der Gruppe bestehend aus H, N(Ar⁵)₂, einer geradkettigen Alkylgruppe mit 1 bis 6 C-Atomen, insbesondere mit 1 bis 4 C-Atomen, oder einer verzweigten oder cyclischen Alkylgruppe mit 3 bis 8 C-Atomen, insbesondere mit 3 bis 6 C-Atomen, die jeweils mit einem oder mehreren Resten R¹ substituiert sein kann, bevorzugt aber unsubstiutiert ist, oder einem aromatischen oder heteroaromatischen Ringsystem mit 5 bis 24 aromatischen Ringatomen, das jeweils mit einem oder mehreren Resten R¹ substituiert sein kann.

Wenn R für ein aromatisches oder heteroaromatisches Ringsystem steht, dann ist dieser Rest R bevorzugt gleich oder verschieden bei jedem Auftreten aus denselben Gruppen ausgewählt, wie unten als geeignete Gruppen für Ar¹ bis Ar⁴ angegeben.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

In einer Ausführungsform der Erfindung ist n = 1 und m = 0. In einer weiteren Ausführungsform der Erfindung ist n = 0 und m = 1. In nochmals einer weiteren Ausführungsform der Erfindung ist n = m = 1. In nochmals einer weiteren Ausführungsform der Erfindung ist n = m = 0.

Die Verbindungen gemäß Formel (1) und (2) enthalten erfindungsgemäß eine Sechsring-Heteroarylgruppe der folgenden Formel, welche im Folgenden als (Het-Ar) abgekürzt wird:

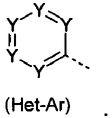

Diese Gruppe ist für m = 1 an Ar¹ bzw. für m = 0 an den Stickstoff gebunden. In der Gruppe (Het-Ar) steht mindestens eine Gruppe Y für N. In einer bevorzugten Ausführungsform der Erfindung stehen ein, zwei oder drei Symbole Y für N und die verbleibenden Symbole Y stehen für CR.

Bevorzugte Ausführungsformen sind die Gruppen der folgenden Formeln (Het-Ar-1) bis (Het-Ar-10), 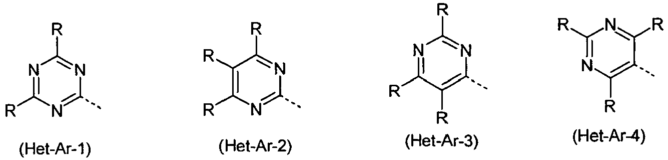 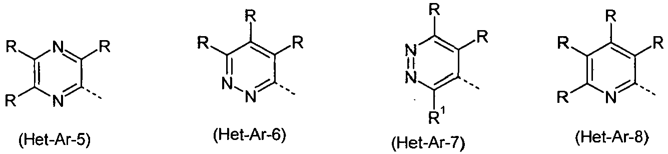 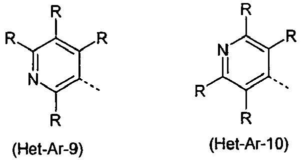 wobei die gestrichelte Bindung die Bindung an Ar¹ bzw. für m = 0 die Bindung an den Stickstoff darstellt und R die oben genannten Bedeutungen aufweist.

Besonders bevorzugt sind die Gruppen der folgenden Formeln (Het-Ar-1a) bis (Het-Ar-10b), 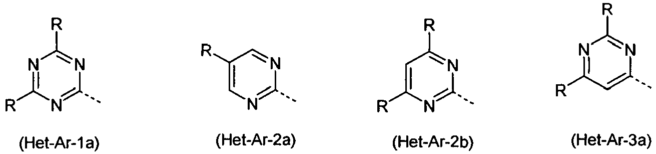 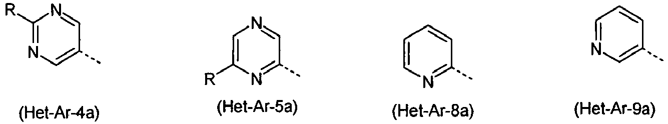 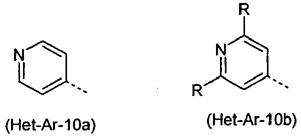 wobei die gestrichelte Bindung die Bindung an Ar¹ bzw. für m = 0 die Bindung an den Stickstoff darstellt und die verwendeten Symbole die oben genannten Bedeutungen aufweisen.

Ganz besonders bevorzugt sind die Gruppen (Het-Ar-1a), (Het-Ar-2a) und (Het-Ar-2b).

In den Gruppen (Het-Ar-1) bis (Het-Ar-10) steht R bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R¹ substituiert sein kann, insbesondere für H oder für Phenyl, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Dibenzofuranyl, insbesondere 1-, 2-, 3- oder 4-Dibenzofuranyl oder Carbazolyl, insbesondere 1-, 2-, 3- oder 4-Carbazolyl, welche jeweils durch einen oder mehrere Reste R¹ substituiert sein können, bevorzugt aber unsubstituiert sind.

In den Gruppen (Het-Ar-1a) bis (Het-Ar-10a) stehen die Substituenten R bevorzugt für ein aromatisches oder heteroaromatisches Ringsystem mit 5 bis 24 aromatischen Ringatomen, welches durch einen oder mehrere Reste R¹ substituiert sein kann, insbesondere für Phenyl, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta-, para- oder verzweigtes Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Dibenzofuranyl, insbesondere 1-, 2-, 3- oder 4-Dibenzofuran oder Carbazolyl, insbesondere 1-, 2-, 3- oder 4-Carbazol, welche jeweils durch einen oder mehrere Reste R¹ substituiert sein können, bevorzugt aber unsubstituiert sind.

In einer bevorzugten Ausführungsform der Erfindung ist m = 1 und Ar¹ ist ein aromatisches oder heteroaromatisches Ringsystem mit 5 bis 24 aromatischen Ringatomen, bevorzugt ein aromatisches Ringsystem mit 6 bis 24 aromatischen Ringatomen, besonders bevorzugt mit 6 bis 12 aromatischen Ringatomen, welches jeweils durch einen oder mehrere Reste R substituiert sein kann. Geeignete Gruppen Ar¹ sind ausgewählt aus Phenylen, insbesondere ortho-, meta- oder para-Phenylen, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 2,7-verknüpftem Fluoren, Spirobifluoren, insbesondere 2,7- oder 2,2'- oder 4,4'-verknüpftem Spirobifluoren, Naphthalin, insbesondere 1,4- oder 2,6-verknüpftem Naphthalin, Indol, Benzofuran, Benzothiophen, Carbazol, insbesondere 2,7- oder 3,6-verknüpftem Carbazol, Dibenzofuran, Dibenzothiophen, Indenocarbazol, Indolocarbazol, Pyridin, Pyrimidin, Pyrazin, Pyridazin, Triazin, Phenanthren, Triphenylen oder Kombinationen aus zwei oder drei dieser Gruppen, welche jeweils mit einem oder mehreren Resten R substituiert sein können. Besonders bevorzugt steht Ar¹ für ein aromatisches Ringsystem, ausgewählt aus der Gruppe bestehend aus Phenylen, insbesondere ortho-, meta- oder para-Phenylen, Biphenyl, insbesondere ortho-, meta- oder para-Biphenyl oder Terphenyl, insbesondere ortho-, meta-, para- oder verzweigtem Terphenyl. Dabei ist die Gruppe (Het-Ar) an einer beliebigen Stelle an Ar¹ gebunden.

In einer weiteren bevorzugten Ausführungsform der Erfindung ist m = 0, und die Gruppe Ar¹ ist nicht vorhanden.

In einer weiteren bevorzugten Ausführungsform der Erfindung sind die aromatischen Gruppen in der Gruppe Ar¹ nicht para-verknüpft, d. h. es handelt sich bevorzugt nicht um para-Phenylen, para-Biphenyl, para-Terphenyl oder para-Quaterphenyl, sondern beispielsweise um die jeweiligen ortho- oder meta-verknüpften Strukturen.

Erfindungsgemäß enthalten die Verbindungen der Formel (1) und (2) in para-Position zum Stickstoffatom der Spirocarbazol-Einheit eine Gruppe der Formel -[Ar²]ₙ-N(Ar³)(Ar⁴).

Dabei können Ar² und Ar³ für n = 1 miteinander und/oder Ar³ und Ar⁴ miteinander auch durch eine Einfachbindung oder durch eine Gruppe ausgewählt aus C(R¹)₂, C(R¹)₂-C(R¹)₂, NR¹, O oder S verbunden sein, bevorzugt durch eine Einfachbindung. Bevorzugt erfolgt die Verknüpfung von Ar² und Ar³ miteinander bzw. von Ar³ und Ar⁴ miteinander jeweils ortho zur Position der Verknüpfung mit dem Stickstoffatom.

In einer Ausführungsform der Erfindung ist n = 1, und Ar² und Ar³ sind miteinander durch eine Einfachbindung verbunden. In einer weiteren Ausführungsform der Erfindung ist n = 0 oder 1, und Ar³ und Ar⁴ sind miteinander durch eine Einfachbindung verbunden. In nochmals einer weiteren Ausführungsform der Erfindung ist n = 0 oder 1, und keine der Gruppen Ar², Ar³ bzw. Ar⁴ sind miteinander verbunden.

Besonders bevorzugt ist n = 1, und Ar² und Ar³ sind miteinander durch eine Einfachbindung verbunden.

Bevorzugt ist Ar² ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R substituiert sein kann. Besonders bevorzugt ist Ar² ausgewählt aus der Gruppe bestehend aus Phenylen, insbesondere ortho-, meta- oder para-Phenylen, oder Biphenyl, insbesondere ortho-, meta- oder para-Biphenyl, welche jeweils durch einen oder mehrere Reste R substituiert sein können, bevorzugt aber unsubstituiert sind. Ganz besonders bevorzugt ist Ar² eine unsubstituierte Phenylengruppe. Dies gilt insbesondere, wenn Ar² mit Ar³ durch einen Einfachbindung verbunden ist.

Bevorzugt sind Ar³ und Ar⁴ gleich oder verschieden bei jedem Auftreten ein aromatisches oder heteroaromatisches Ringsystem mit 6 bis 24 aromatischen Ringatomen, das jeweils mit einem oder mehreren Resten R substituiert sein kann. Besonders bevorzugte Gruppen Ar³ bzw. Ar⁴ sind gleich oder verschieden bei jedem Auftreten ausgewählt aus der Gruppe bestehend aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluorenyl, insbesondere 1-, 2-, 3- oder 4-Fluorenyl, Spirobifluorenyl, insbesondere 1-, 2-, 3- oder 4-Spirobifluorenyl, Naphthyl, insbesondere 1- oder 2-Naphthyl, Indolyl, Benzofuranyl, Benzothiophenyl, Carbazolyl, insbesondere 1-, 2-, 3- oder 4-Carbazolyl, Dibenzofuranyl, insbesondere 1-, 2-, 3- oder 4-Dibenzofuranyl, Dibenzothiophenyl, insbesondere 1-, 2-, 3- oder 4-Dibenzothiophenyl, Indenocarbazolyl, Indolocarbazolyl, Pyridinyl, insbesondere 2-, 3- oder 4-Pyridinyl, Pyrimidinyl, insbesondere 2-, 4- oder 5-Pyrimidinyl, Pyrazinyl, Pyridazinyl, Triazinyl, Phenanthrenyl, Triphenylenyl oder Kombinationen aus zwei, drei oder vier dieser Gruppen, welche jeweils mit einem oder mehreren Resten R substituiert sein können. Besonders bevorzugt stehen Ar³ und Ar⁴ gleich oder verschieden bei jedem Auftreten für ein aromatisches Ringsystem mit 6 bis 24 aromatischen Ringatomen, welches durch einen oder mehrere Reste R substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluorenyl, insbesondere 1-, 2-, 3- oder 4-Fluorenyl, oder Spirobifluorenyl, insbesondere 1-, 2-, 3- oder 4-Spirobifluorenyl.

Besonders bevorzugte Gruppen der Formel -[Ar²]ₙ-N(Ar³)(Ar⁴) sind solche, in denen n = 1 ist und Ar² für eine Phenylgruppe steht, die mit Ar³ durch eine Einfachbindung verbunden ist. Dadurch entsteht ein Carbazol bzw. ein Carbazolderivat.

Besonders bevorzugte Gruppen der Formel -[Ar²]ₙ-N(Ar³)(Ar⁴) sind die Gruppen der folgenden Formel (CARB), 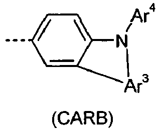 wobei Ar³ und Ar⁴ die oben genannten Bedeutungen und insbesondere die oben genannten bevorzugten Bedeutungen aufweisen und die gestrichlte Bindung die Bindung der Gruppe (CARB) an das Spirocarbazol-Grundgerüst in Formel (1) bzw. Formel (2) darstellt.

Bevorzugte Ausführungsformen der Formel (CARB) sind die Gruppen der folgenden Formeln (CARB-1) bis (CARB-5), 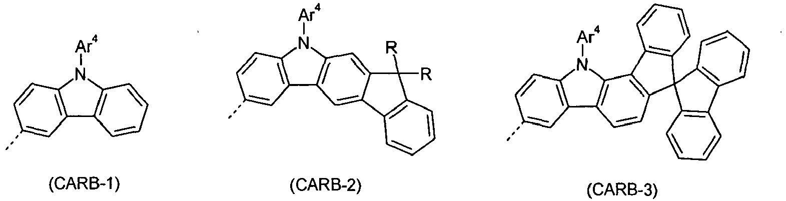 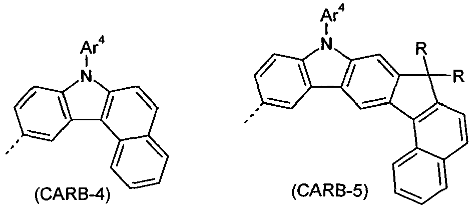 wobei Ar⁴ die oben genannten Bedeutungen aufweist und die Strukturen durch einen oder mehrere Reste R substituiert sein können.

Dabei sind die in Formel (CARB-2) und (CARB-4) explizit eingezeichneten Reste R bevorzugt ausgewählt aus Alkylgruppen mit 1 bis 4 C-Atomen oder aromatischen Ringsystemen mit 6 bis 12 aromatischen Ringatomen, insbesondere Methyl.

Wenn die Verbindungen der Formel (1) oder (2) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Triplettenergie der Verbindungen gleich hoch oder höher ist als die des phosphoreszierenden Emitters. Dies kann insbesondere für grün und blau phosphoreszierende Emitter, welche eine höhere Triplettenergie als rot phosphoreszierende Emitter aufweisen, dadurch erreicht werden, dass die erfindungsgemäße Verbindung keine kondensierten Aryl- bzw. Heteroarylgruppen enthält, in denen mehr als zwei Sechsringe direkt aneinander ankondensiert sind. Solche Verbindungen sind daher eine weitere bevorzugte Ausführungsform der Erfindung. Insbesondere ist es für diese Verwendung bevorzugt, dass die Reste R, R¹ und Ar¹ bis Ar⁴ keine kondensierten Aryl- bzw. Heteroarylgruppen enthalten, in denen zwei oder mehr Sechsringe direkt aneinander ankondensiert sind.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

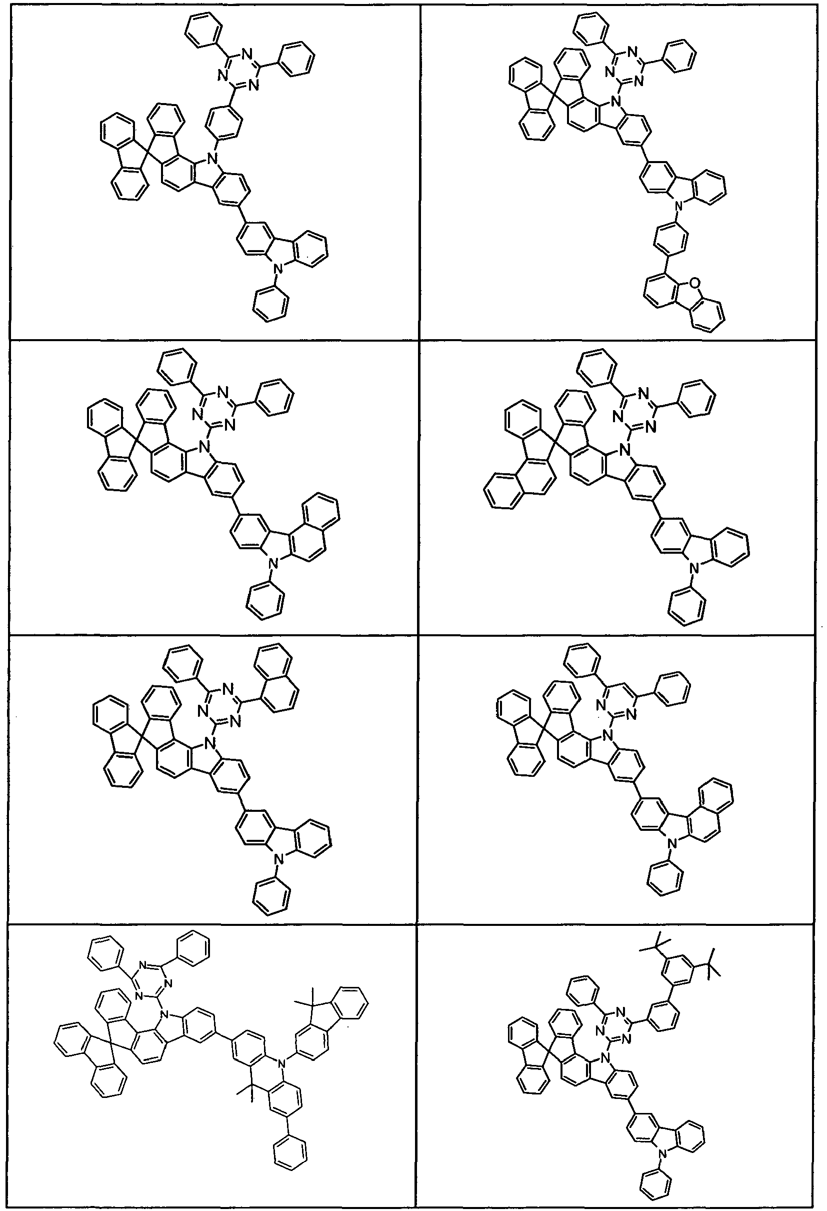

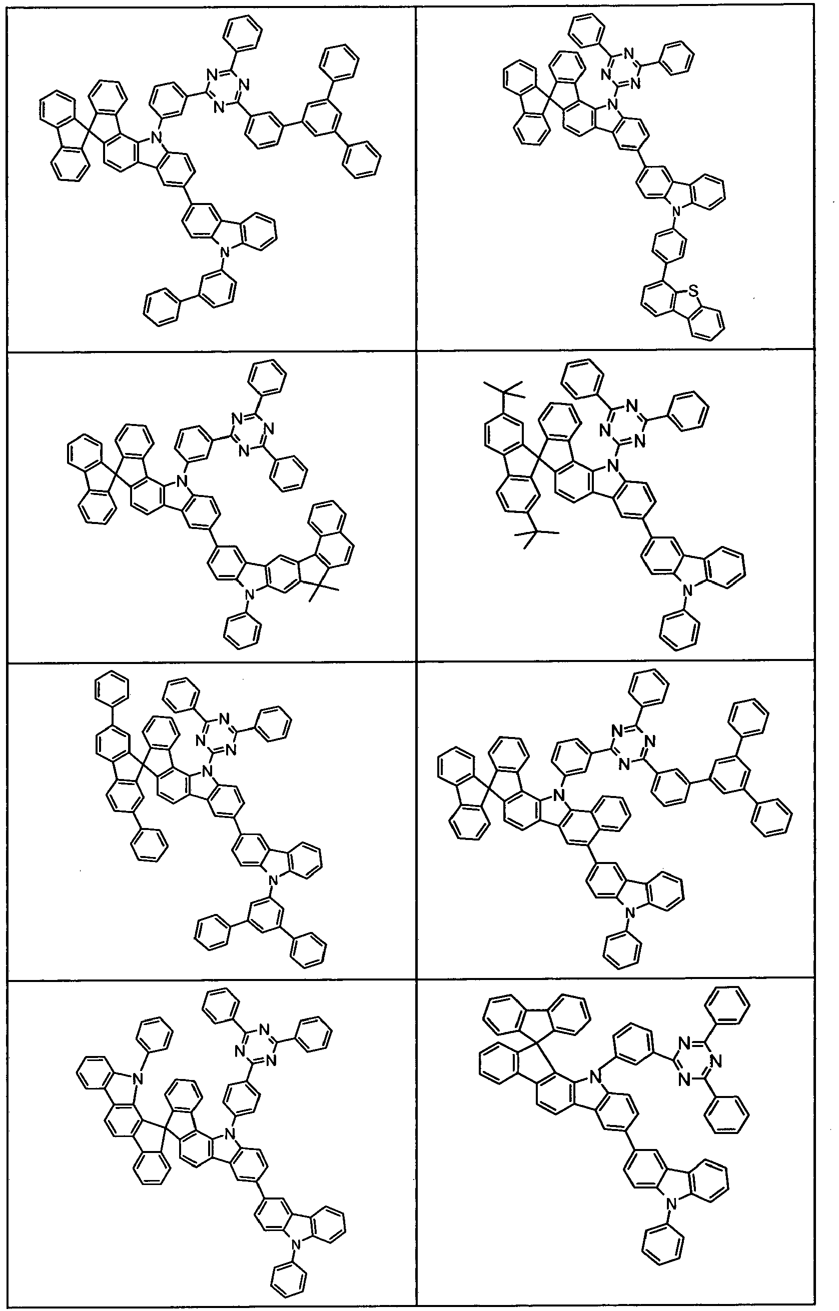

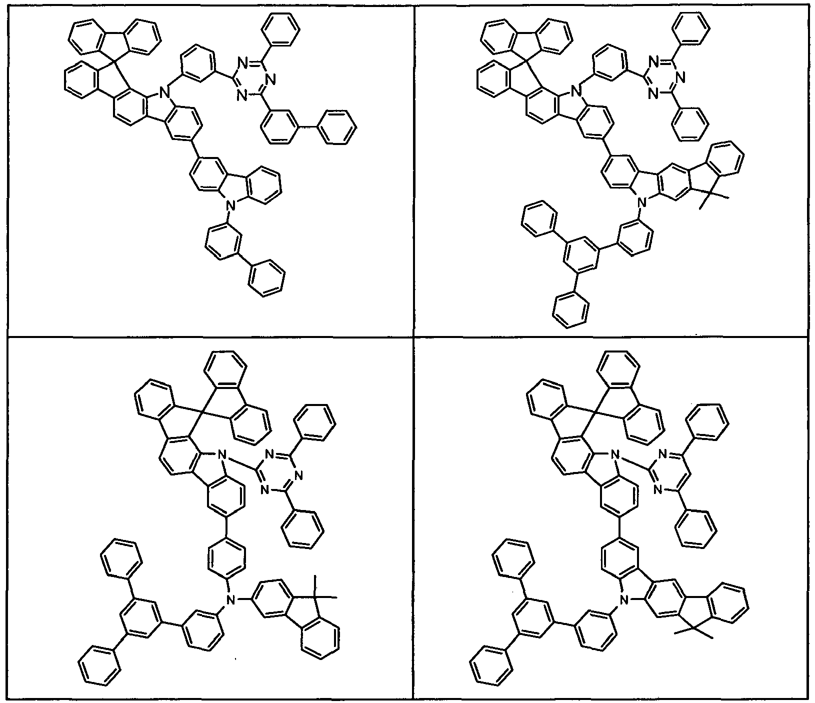

Die Grundstrukturen der erfindungsgemäßen Verbindungen können auf den in den Schemata 1a und 1b skizzierten Wegen hergestellt werden. Weitere Derivatisierung kann gemäß der Schemata 2 und/oder 3 erfolgen.

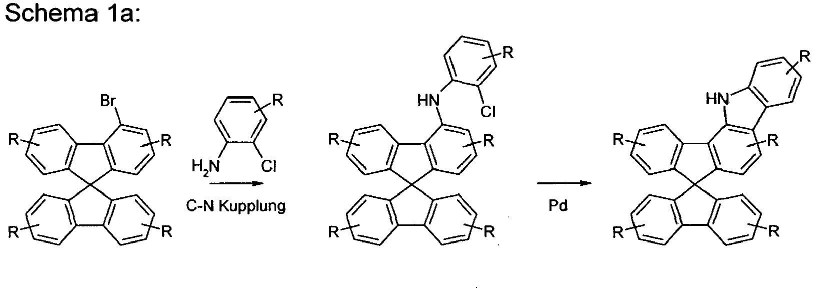

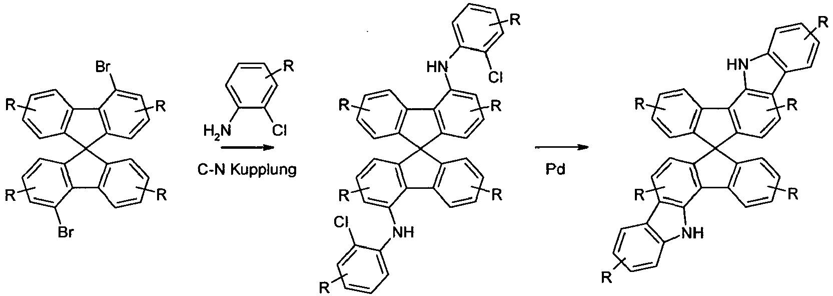

Die Synthese der Verbindungen der Formel (1) erfolgt üblicherweise ausgehend von den literaturbekannten Verbindungen 4-Brom-9,9'-spirobifluoren (Org. Lett. 2009, 11, 2607) bzw. 4,4'-Dibrom-9,9'-spirobifluoren (Org. Lett. 2010, 12, 5648) oder entsprechend substituierter Derivate. Diese werden in einer C-N Kupplungsreaktion, beispielsweise unter Pd- oder Cu-Katalyse, mit einem ortho-Halogenaminobenzol umgesetzt, wobei das Halogen bevorzugt Cl, Br oder I ist. Anschließender Ringschluss durch eine intramolekulare Pd-katalysierte Kupplungsreaktion führt zu Verbindungen der Formel (1).

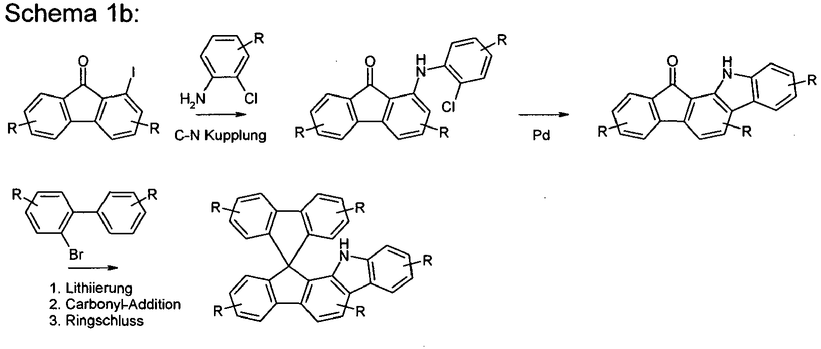

Die Synthese der Verbindungen der Formel (2) erfolgt üblicherweise ausgehend von der literaturbekannten Verbindung 1-Iod-9-fluorenon (Tetrahedron Lett. 2002, 43, 8347) oder entsprechend substituierter Derivate. Diese wird in einer C-N Kupplungsreaktion, beispielsweise unter Pd- oder Cu-Katalyse, mit einem ortho-Halogenaminobenzol umgesetzt, wobei das Halogen bevorzugt Cl, Br oder I ist. Anschließender Ringschluss durch eine intramolekulare Pd-katalysierte Kupplungsreaktion führt zum Indolocarbazol-Derivat. Im letzten Schritt erfolgt eine Ringschlussreaktion zum Spirobifluoren-Derivat durch Umsetzung mit einem in 2-Position lithiierten Biphenylderivat und abschließender saurer Cyclisierung.

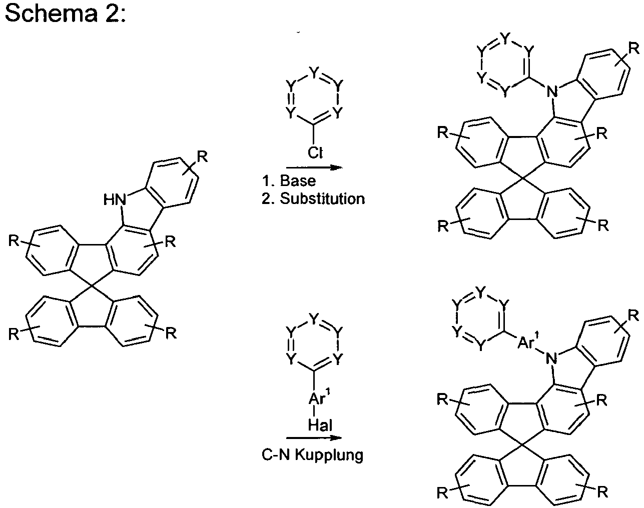

Vorstufen der Verbindungen der Formel (1) mit m = 0, die noch keine Amino- bzw. Carbazolgruppe als Substituent enthalten, erhält man durch nukleophile aromatische Substitution mit einer Gruppe (Het-Ar)-Hal, wobei das Halogen als Abgangsgruppe bevorzugt Cl oder Br ist.

Vorstufen der Verbindungen der Formel (1) mit m = 1, die noch keine Amino- bzw. Carbazolgruppe als Substituent enthalten, erhält man durch C-N Kupplungsreaktionen mit einer Gruppe (Het-Ar)-Ar¹-Hal, beispielsweise Hartwig-Buchwald-Kupplung oder Ullmann-Kupplung, wobei das Halogen bevorzugt Br oder I ist.

Die in Schema 2 beschriebenen Reaktionen können in analoger Weise auch mit Indolocarbazol-Derivaten nach Schema 1b durchgeführt werden und führen zu den entsprechenden Vorstufen der Verbindungen der Formel (2).

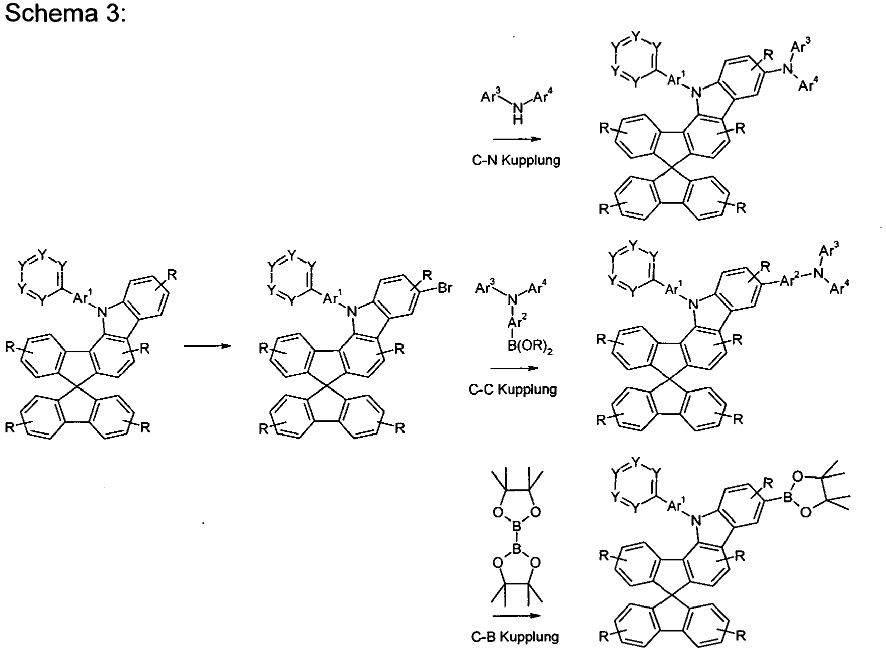

Die weitere Derivatisierung kann durch Bromierung der Carbazol-Derivate erfolgen, beispielsweise durch Umsetzung mit *N*-Bromsuccinimid oder elementarem Brom. Anschließende Pd-katalysierte C-N Kupplungsreaktionen, beispielsweise Hartwig-Buchwald- oder Ullmann-Kupplung, bzw. C-C Kupplungsreaktionen, beispielsweise Suzuki-, Negishi-, Yamamoto-, Grignard-Cross- oder Stille-Kupplung, führen zu erfindungsgemäßen Verbindungen gemäß Formel (1) mit n = 0 bzw. n = 1. Analog kann auch eine Umsetzung mit einem Boronsäurederivat eines unsubstituierten Carbazols erfolgen, also beispielsweise mit einer Verbindung Ar³-NH-Ar²-B(OR)₂, wobei das Proton am Carbazol in einem weiteren Schritt substituiert wird. Weiterhin können aus dem Bromid in einer Pd-katalysierten Umsetzung beispielsweise mit Bis(pinacolato)diboran entsprechende Boronsäureester dargestellt werden, welche in einer C-C Kupplungsreaktion umgesetzt werden können.

Die beschriebene Bromierung und nachfolgende Umsetzung zu Boronsäureestern können in analoger Weise auch in Abwesenheit der Gruppe Het-Ar-Ar¹ bzw. Het-Ar durchgeführt werden, so dass diese Gruppe auch erst im letzten Schritt eingeführt werden kann.

Die beschriebenen Reaktionen können in analoger Weise auch mit Derivaten nach Schema 1b bzw. deren nach Schema 2 *N*-substituierten Derivaten durchgeführt werden, wobei C-N bzw. C-C Kupplungsreaktionen die erfindungsgemäßen Verbindungen gemäß Formel (2) liefern.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrix-material für phosphoreszierende Emitter in einer emittierenden Schicht. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Gew.-%, vorzugsweise zwischen 98 und 10 Gew.-%, besonders bevorzugt zwischen 97 und 60 Gew.-%, insbesondere zwischen 95 und 80 Gew.-% der erfindungsgemäßen Verbindung bezogen auf die Gesamtmischung aus Emitter und Matrixmaterial. Entsprechend enthält die Mischung zwischen 1 und 99 Gew.-%, vorzugsweise zwischen 2 und 90 Gew.-%, besonders bevorzugt zwischen 3 und 40 Gew.-%, insbesondere zwischen 5 und 30 Gew.-% des Emitters bezogen auf die Gesamtmischung aus Emitter und Matrix-material.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Wenn die erfindungsgemäße Verbindung in Kombination mit einem weiteren Matrixmaterial eingesetzt wird, liegt deren Anteil bevorzugt bei 20 bis 50 Gew.-%, bezogen auf die Gesamtmischung. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/044988, WO 2011/157339, WO 2012/007086, WO 2012/163471, WO 2013/000531 und WO 2013/020631, WO 2014/008982, WO 2014/023377 entnommen werden. Weiterhin eignen sich beispielsweise die in den nicht offen gelegten Anmeldungen EP 12008582.4, EP 13003484.6, EP 13003485.3, EP 13004552.9, EP 14000345.0 und EP 14000417.7 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren aufgebracht werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation aufgebracht werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Bei Verarbeitung aus Lösung weisen die erfindungsgemäßen Verbindungen sehr gute Filmbildungseigenschaften auf, charakterisiert durch gute Löslichkeit sowie hohe Glasübergangstemperaturen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### Beispiel 1a: Synthese von N-(9,9-Dimethylfluoren-2-yl)-4-aminobiphenyl

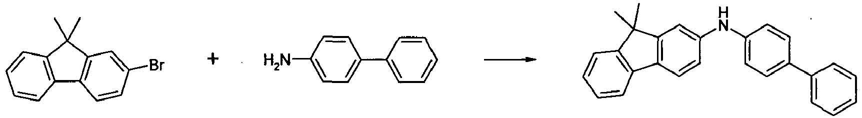

24.0 g (142 mmol) 4-Aminobiphenyl [92-67-1] und 32.0 g (117 mmol) 2-Brom-9,9'-dimethylfluoren [28320-31-2] werden in 950 ml Toluol vorgelegt. Das Gemisch wird unter kräftigem Rühren für 30 Minuten mit Argon durchspült. 1.0 g (1.8 mmol) 1,1'-Bis(diphenylphosphino)ferrocen [12150-46-8], 355 mg (1.6 mmol) Palladium(II)acetat und 28.8 g (300 mmol) Natrium-tert-butylat werden zugegeben und die Reaktionsmischung für 15 h unter Rückfluss erhitzt. Nach Abkühlen auf Raumtemperatur wird das Reaktionsgemisch mit 300 ml Toluol und 1200 ml Wasser erweitert. Die organische Phase wird abgetrennt, dreimal mit je 250 ml Wasser gewaschen und über Natriumsulfat getrocknet. Das Lösungsmittel wird am Rotationsverdampfer entfernt. Das verbleibende Öl wird mit 50 ml Essigester versetzt und langsam in 800 ml einer Heptan/Essigester-Mischung (20:1) eingerührt. Der gebildete Feststoff wird abgesaugt, zweimal mit etwa 50 ml Heptan gewaschen und im Vakuum getrocknet. Es verbleiben 29.2 g (80 mmol, 69% der Theorie) des Produkts mit einer Reinheit von 99% nach HPLC.

In analoger Weise kann folgende Verbindung hergestellt werden:

| Bsp. | Edukt | Produkt | Ausbeute |
|---|---|---|---|
| 1b | 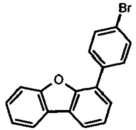 | 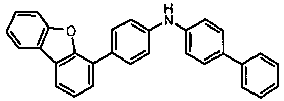 | 74% |
| | [955959-84-9] | | |

### Beispiel 2a: Synthese von N-(Biphenyl-2-yl)-N-(biphenyl-4-yl)-4-(4,4,5,5-tetramethyl-[1,3,2]dioxaborolan-2-yl)-phenylamin

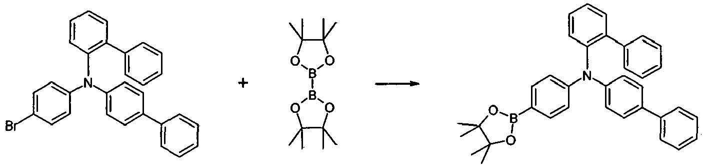

33.8 g (71 mmol) *N*-(Biphenyl-2-yl)-*N*-(biphenyl-4-yl)-*N*-(4-bromphenyl)-amin [1371651-92-1], 21.9 g Bis(pinacolato)diboran (86 mmol) [73183-34-3], 21.7 g (221 mmol) Kaliumacetat und 1.7 g (2.1 mmol) 1,1'-Bis(diphenyl-phosphino)ferrocen-dichloropalladium(II)-Dichlormethan-Addukt [95464-26-4] werden in 1000 ml wasserfreiem Dioxan für 16 h unter Rückfluss erhitzt. Nach Abkühlen auf Raumtemperatur wird die organische Phase mit 750 ml Essigester erweitert, dreimal mit je 300 ml Wasser gewaschen und über Natriumsulfat getrocknet. Das Lösungsmittel wird am Rotationsverdampfer entfernt und der Rückstand zweimal aus Heptan umkristallisiert. Es verbleiben 22.6 g (43 mmol, 61% der Theorie) des Produkts als schwach gelber Feststoff mit einer Reinheit von etwa 99% nach ¹H-NMR.

### Beispiel 3a: Synthese von 2-(Biphenyl-3-yl)-4-chlor-6-phenyl-[1,3,5]-triazin

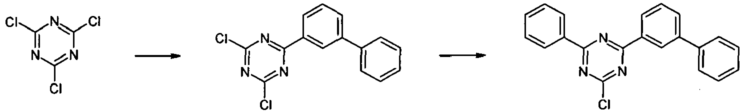

11.6 g (477 mmol) Magnesium werden mit einem Körnchen Iod aktiviert. 30 ml einer Lösung von 100 g (429 mmol) 3-Brombiphenyl [2113-57-7] in 800 ml THF werden zugegeben; die Reaktion wird mit einem Heißluftfön gestartet. Nach Einsetzen der Reaktion wird die restliche Lösung so zugetropft, dass sich der Rückfluss durch die Reaktionswärme hält. Nach vollständiger Zugabe wird die Reaktionsmischung noch 2 h unter Rückfluss erhitzt.

79.1 g (429 mmol) 2,4,6-Trichlor-[1,3,5]-triazin werden in 500 ml THF vorgelegt und auf -5 °C abgekühlt. Obige Grignard-Lösung wird so zugetropft, dass die Innentemperatur 0 °C nicht übersteigt. Die Kühlung wird entfernt und das Gemisch für 16 h gerührt; anschließend wird erneut auf -5 °C abgekühlt und 219 ml (438 mmol) Phenylmagnesiumchlorid-Lösung (2M in THF) werden so zugetropft, dass die Innentemperatur 0 °C nicht übersteigt. Die Kühlung wird entfernt und das Gemisch für 18 h gerührt. 450 ml 1M Salzsäure werden langsam eingerührt. Nach 1 h wird der gebildete Feststoff abgesaugt und im Vakuum getrocknet. Nach zweimaliger Umkristallisation aus Toluol verbleiben 52.9 g (154 mmol, 36% der Theorie) des Produkts als hellbrauner Feststoff mit einer Reinheit von etwa 98% nach ¹H-NMR.

In analoger Weise können folgende Verbindungen hergestellt werden:

| Bsp. | Edukt Stufe 1 | Produkt | Ausbeute |
|---|---|---|---|
| 3b | 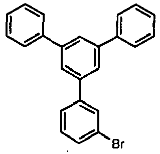 | 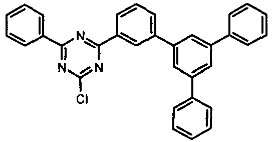 | 31% |
| | [1233200-57-1] | | |
| 3c | 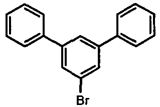 | 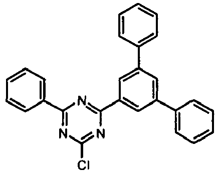 | 37% |
| | [103068-20-8] | | |

### Beispiel 4a: Synthese von 10-(3-Bromphenyl)-12,12-dimethyl-10,12-dihydro-10-aza-indeno[2,1-b]fluoren

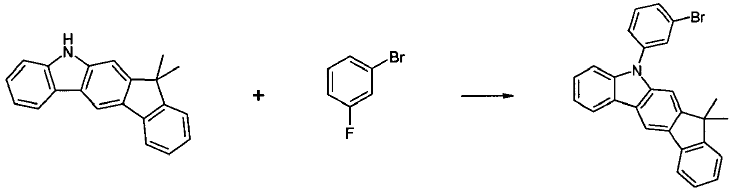

150.0 g (526 mmol) 12,12-Dimethyl-10,12-dihydro-10-aza-indeno[2,1-b]fluoren [1257220-47-5], 184.0 g (1.05 mol) 1-Brom-3-fluorbenzol [1073-06-9] und 334.7 g (1.58 mol) Kaliumphosphat werden in 2 l Dimethylacetamid vorgelegt und für 14 h unter Rückfluss erhitzt. Nach Abkühlen auf Raumtemperatur wird das Lösungsmittel am Rotationsverdampfer so weit wie möglich entfernt; es verbleibt ein dunkelbraunes Öl. Nach kräftigem Anreiben der Kolbenwand mit einem Glasstab kann das Produkt durch langsames Einrühren von 750 ml Ethanol ausgefällt werden. Der gebildete Feststoff wird abgesaugt, viermal mit je 250 ml Ethanol gewaschen, im Vakuum getrocknet und abschließend bei einem Druck von etwa 10⁻⁵ mbar und 220 °C sublimiert. Es verbleiben 152.2 g (347 mmol, 66% der Theorie) des Produkts als gelber glasartiger Feststoff mit einer Reinheit von etwa 99% nach ¹H-NMR.

### Beispiel 5a: Synthese von (2-Chlorphenyl)-(spiro-9,9'-bifluoren-4-yl)-amin

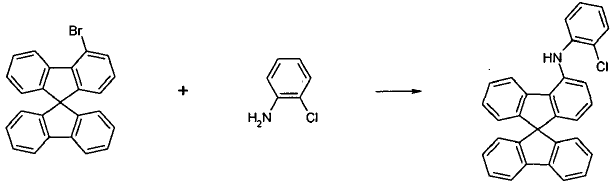

54.1 g (137 mmol) 4-Bromspiro-9,9'-bifluoren [1161009-88-6], 17.9 g (140 mmol) 2-Chloranilin [95-51-2], 68.2 g (710 mmol) Natrium-*tert*-butylat, 613 mg (2.7 mmol) Palladium(II)acetat und 3.03 g (5.5 mmol) 1,1'-Bis(di-phenylphosphino)ferrocen werden in 1300 ml Toluol vorgelegt und für 5 h unter Rückfluss erhitzt. Nach Abkühlen auf Raumtemperatur wird das Reaktionsgemisch mit 700 ml Toluol erweitert und über Celite filtriert. Das Lösungsmittel wird am Rotationsverdampfer entfernt und der Rückstand aus einer Toluol/Heptan-Mischung (1:2) umkristallisiert. Nach Trocknen im Vakuum verbleiben 52.2 g (118 mmol, 86% der Theorie) des Produkts als hellgelber Feststoff.

In analoger Weise kann folgende Verbindung hergestellt werden:

| Bsp. | Edukt | Produkt | Ausbeute |
|---|---|---|---|
| 5b | 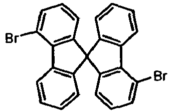 | 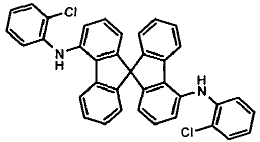 | 72% |
| | [1257321-41-7] | | |

### Beispiel 6a: 1-(2-Chlorphenylamin)-fluoren-9-on

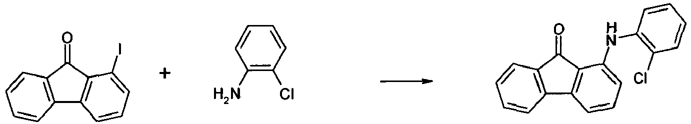

52 g (166 mmol) 1-Iodfluoren-9-on [52086-21-2], 19.0 ml (171 mmol) 2-Chloranilin [95-51-2], 59.8 g (432 mmol) Kaliumcarbonat, 3.85 g (6.6 mmol) 1,1'-(9,9-Dimethyl-9*H*-xanthen-4,5-diyl)-bis(1,1-diphenyl)-phosphin [161265-03-8] und 746 mg (3.3 mmol) Palladium(II)acetat werden in 400 ml Toluol vorgelegt und 15 h unter Rückfluss erhitzt. Nach Abkühlen auf Raumtemperatur wird mit 200 ml Toluol und 500 ml Wasser erweitert; die organische Phase wird abgetrennt, zweimal mit je 200 ml 3M Salzsäure und zweimal mit je 200 ml Wasser gewaschen und über eine dünne Schicht Aluminiumoxid (basisch, Aktivitätsstufe 1) filtriert. Das Lösungsmittel wird am Rotationsverdampfer entfernt. Nach Trocknen im Vakuum verbleiben 48.0 g (157 mmol, 95% der Theorie) des Produkts als orangefarbener Feststoff mit einer Reinheit von etwa 97% nach ¹H-NMR.

### Beispiel 7a: Synthese von Spiro[9H-fluoren-9,7'(1'H)-indeno[1,2-a]carbazol]

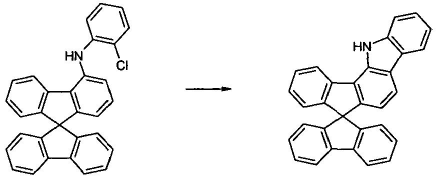

45.0 g (102 mmol) (2-Chlorphenyl)-(spiro-9,9'-bifluoren-4-yl)-amin (aus Bsp. 5a), 56.0 g (405 mmol) Kaliumcarbonat, 4.5 g (12 mmol) Tricyclohexylphosphoniumtetrafluoroborat und 1.38 g (6 mmol) Palladium(II)acetat werden in 500 ml Dimethylacetamid suspendiert und für 6 h unter Rückfluss erhitzt. Nach Abkühlen auf Raumtemperatur wird die Reaktionsmischung mit 600 ml Dichlormethan und 300 ml Wasser erweitert und 30 Minuten gerührt. Die organische Phase wird abgetrennt und am Rotationsverdampfer vom Lösungsmittel befreit. Der Rückstand wird mit 250 ml Toluol über ein Bett aus Aluminiumoxid (basisch, Aktivitätsstufe 1) heißextrahiert und abschließend einmal aus Toluol umkristallisiert. Es verbleiben 32.5 g (80 mmol, 78% der Theorie) des Produkts als beigefarbener Feststoff mit einer Reinheit von etwa 98% nach ¹H-NMR.

In analoger Weise können folgende Verbindungen hergestellt werden:

| Bsp. | Edukt | Produkt | Ausbeute |
|---|---|---|---|
| 7b | 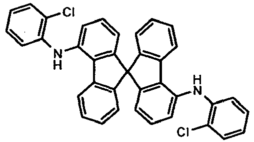 | 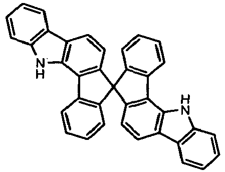 | 68% |
| | Bsp. 5b | | |
| 7c | 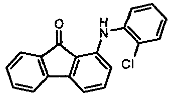 | 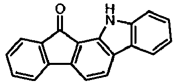 | 87% |
| | Bsp. 6a | | |

### Beispiel 8a: Synthese von N-Phenyl-spiro[9H-fluoren-9,7'(1'H-indeno[1,2-a]carbazol]

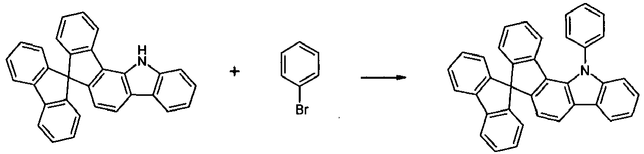

43.0 g (106 mmol) Spiro[9*H*-fluoren-9,7'(1'*H*)-indeno[1,2-*a*]carbazol] (aus Bsp. 7a), 17.9 g (114 mmol) Brombenzol, 30.5 g (317 mmol) Natrium-*tert-*butylat, 0.5 g (2.2 mmol) Palladium(II)acetat und 4.2 ml Tri-*tert*-butyl-phosphin-Lösung (1M in Toluol) werden in 1500 ml *p*-Xylol vorgelegt und für 16 h unter Rückfluss erhitzt. Nach Abkühlen auf Raumtemperatur wird die organische Phase von festen Bestandteilen abgetrennt, dreimal mit je 200 ml Wasser gewaschen und anschließend am Rotationsverdampfer vom Lösungsmittel befreit. Der Rückstand wird mit etwa 300 ml Toluol über Aluminiumoxid (basisch, Aktivitätsstufe 1) heißextrahiert und abschließend zweimal aus Toluol umkristallisiert. Es verbleiben 37.6 g (78 mmol, 74% der Theorie) des Produkts als hellgelber Feststoff mit einer Reinheit von etwa 99% nach ¹H-NMR.

In analoger Weise können folgende Verbindungen hergestellt werden:

| Bsp. | Edukt 1 | Edukt 2 | Produkt | Ausbeute |
|---|---|---|---|---|
| 8b | 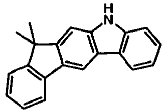 | 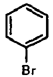 | 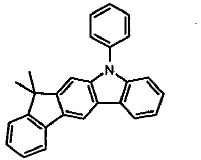 | 83% |
| | [1257220-47-5] | [108-86-1] | | |
| 8c | 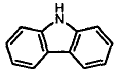 | 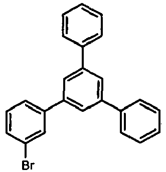 | 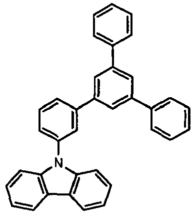 | 77% |
| | [86-74-8] | | | |
| | | [1233200-57-1] | | |
| 8d | 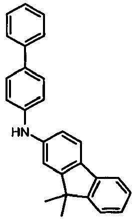 | 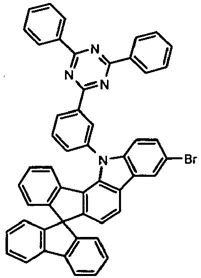 | 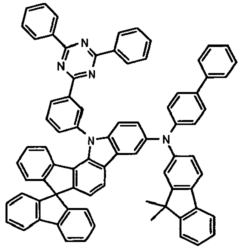 | 51% |
| | Bsp. 1a | Bsp. 10f | | |
| 8e | 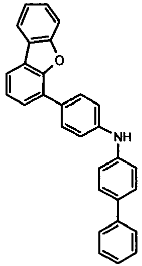 | 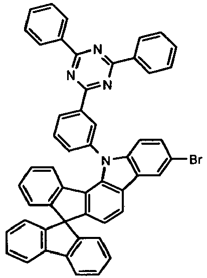 | 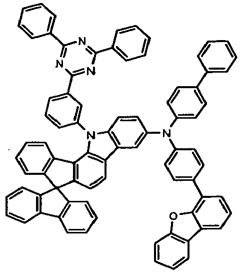 | 54% |
| | Bsp. 1b | Bsp. 10f | | |
| 8f | 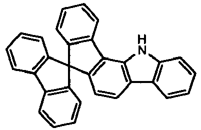 | 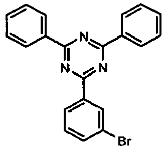 | 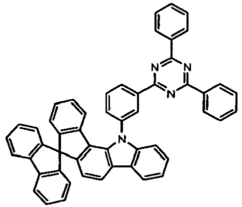 | 53% |
| | Bsp. 7a | [864377-31-1] | | |
| 8g | 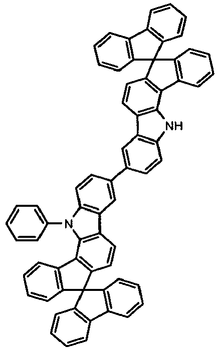 | 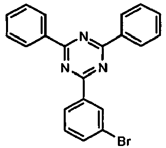 | 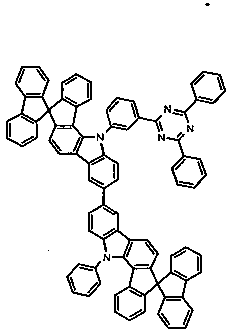 | 61% |
| | | [864377-31-1] | | |
| | Bsp. 12d | | | |
| 8h | 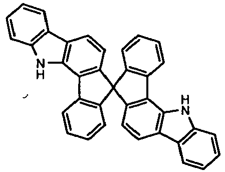 | 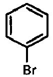 | 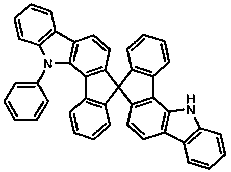 | 82% |
| | Bsp. 7b | [108-86-1] | | |
| 8i | 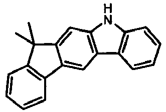 | 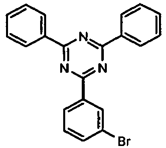 | 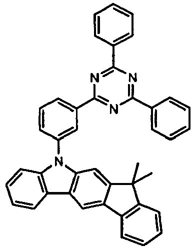 | 58% |
| | [1257220-47-5] | [864377-31-1] | | |
| 8j | 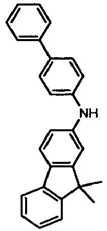 | 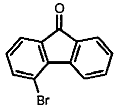 | 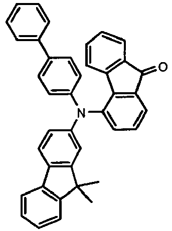 | 77% |
| | | [4269-17-4] | | |
| | Bsp. 1a | | | |
| 8k | 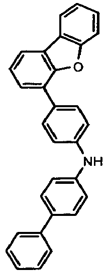 | 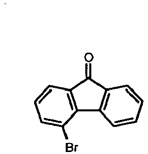 | 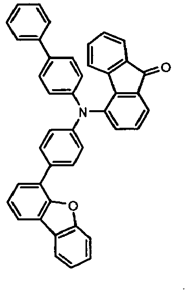 | 67% |
| | | [4269-17-4] | | |
| | Bsp. 1b | | | |

### Beispiel 9a: Synthese von N-(4'-Bromspiro-9,9'-bifluoren-4-yl)-N-(9,9'-dimethylfluoren-2-yl)-4-aminobiphenyl

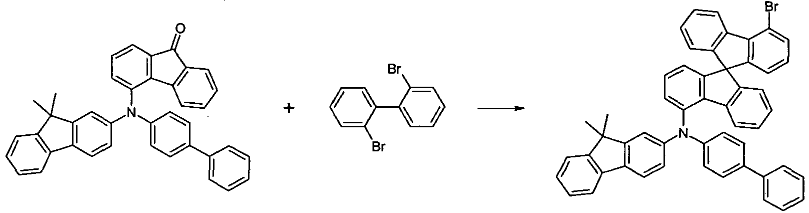

24.6 g (79 mmol) 2,2'-Dibrombiphenyl [13029-09-9] werden in 200 ml THF auf -78 °C gekühlt. 32 ml *n*-Butyllithium (2.5M in Hexan) werden so zugetropft, dass die Innentemperatur -70 °C nicht übersteigt. Die Reaktionsmischung wird 2 h gerührt. Dann wird eine Suspension von 26.9 g (50 mmol) *N*-(9,9-Dimethylfluoren-2-yl)-*N*-(fluorenon-4-yl)-4-aminobiphenyl (aus Bsp. 8j) so zugegeben, dass die Innentemperatur -70 °C nicht übersteigt. Die Kühlung wird entfernt und das Gemisch weitere 14 h gerührt. Nach Zugabe von 100 ml Wasser wird 15 Minuten gerührt, die organische Phase abgetrennt und am Rotationsverdampfer vom Lösungsmittel befreit. Der Rückstand wird in 500 ml Eisessig suspendiert, mit 0.5 ml konzentrierter Schwefelsäure versetzt und für 2 h bei 100 °C gerührt. Nach Abkühlen auf Raumtemperatur wird der gebildete Feststoff abgesaugt, mit 100 ml Eisessig und dreimal mit je 100 ml Ethanol gewaschen und abschließend aus Dioxan umkristallisiert. Nach Trocknen im Vakuum verbleiben 25.5 g (34 mmol, 68% der Theorie) des Produkts als hellroter Feststoff mit einer Reinheit von etwa 98% nach ¹H-NMR.

In analoger Weise können folgende Verbindungen hergestellt werden:

| Bsp. | Edukt | Produkt | Ausbeute |
|---|---|---|---|
| 9b | 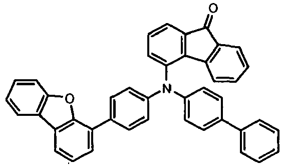 | 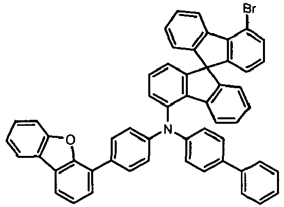 | 69% |
| | Bsp. 8k | | |
| 9c | 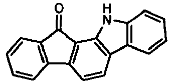 | 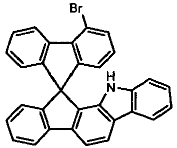 | 83% |
| | Bsp. 7c | | |

In analoger Weise können durch Lithiierung von 2-Brombiphenyl [2052-07-5] in der ersten Stufe folgende Verbindungen hergestellt werden:

| Bsp. | Edukt | Produkt | Ausbeute |
|---|---|---|---|
| 9d | 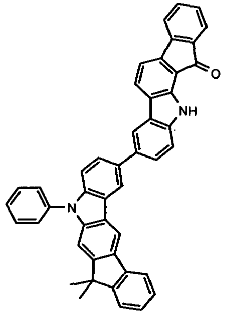 | 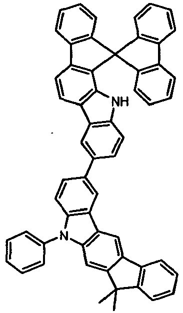 | 74% |
| | Bsp. 12n | | |
| 9e | 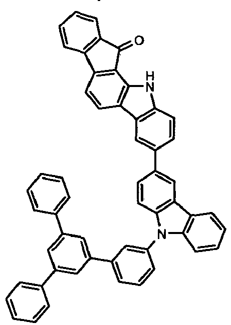 | 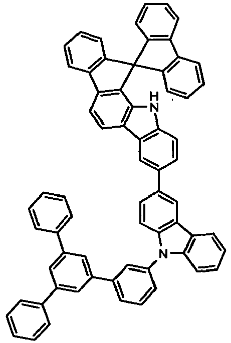 | 70% |
| | Bsp. 12o | | |

### Beispiel 10a: Synthese von N-Phenyl-spiro[9H-fluoren-6'-brom-9,7'(1'H)-indeno[1,2-a]carbazol]

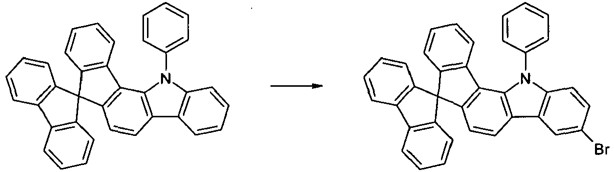

85.2 g (177 mmol) *N*-Phenyl-spiro[9*H*-fluoren-9,7'(1'*H*)-indeno[1,2-*a*]-carbazol] (aus Bsp. 8a) werden in 1500 ml THF vorgelegt. Die Reaktionsmischung wird auf 0 °C gekühlt und innerhalb von 30 Minuten portionsweise mit 31.5 g (177 mmol) *N*-Bromsuccinimid versetzt. Die Kühlung wird entfernt, das Gemisch für 14 h gerührt und anschließend auf etwa 250 ml eingeengt. Unter kräftigem Rühren werden 1000 ml Wasser zugegeben, der gebildete Feststoff wird abgesaugt und zweimal mit je 800 ml Ethanol ausgekocht. Nach Trocknen in Vakuum verbleiben 87.1 g (155 mmol, 88% der Theorie) des Produkts als farbloser Feststoff mit einer Reinheit von etwa 98% nach ¹H-NMR.

In analoger Weise können folgende Verbindungen hergestellt werden:

| Bsp. | Edukt | Produkt | Ausbeute |
|---|---|---|---|
| 10b | 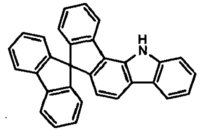 | 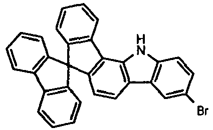 | 60% |
| | Bsp. 7a | | |
| 10c | 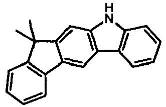 | 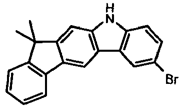 | 65% |
| | [1257220-47-5] | | |
| 10d | 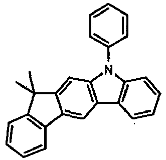 | 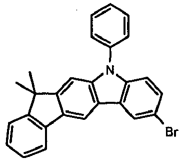 | 81% |
| | Bs. 8b | | |
| 10e | 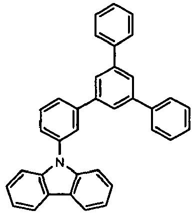 | 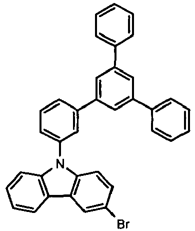 | 81% |
| | Bsp. 8c | | |
| 10f | 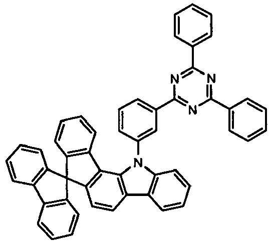 | 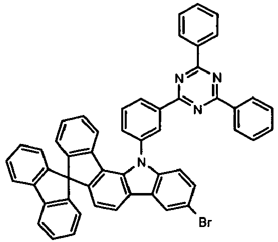 | 49% |
| | Bsp. 8f | | |
| 10g | 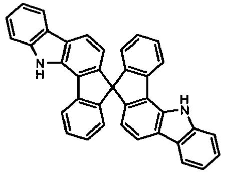 | 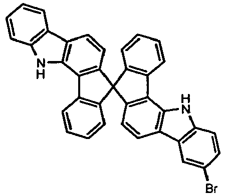 | 52% |
| | Bsp. 7b | | |
| 10h | 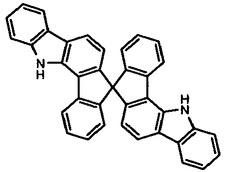 | 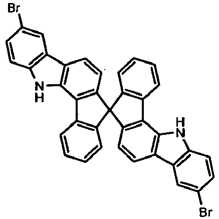 | 61% |
| | Bsp. 7b | | |
| 10i | 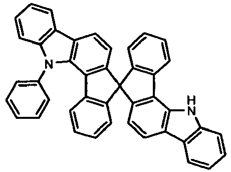 | 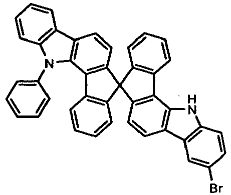 | 12% nach Säulenchromatographie |
| | Bsp. 8h | | |
| 10j | 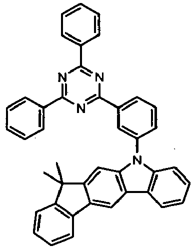 | 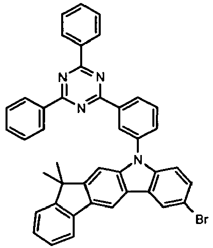 | 68% |
| | Bsp. 8i | | |
| 10k | 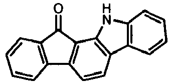 | 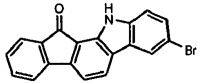 | 90% |
| | Bsp. 7c | | |
| 10l | 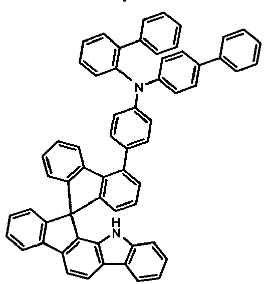 | 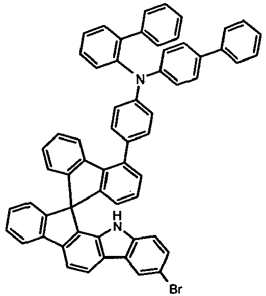 | 51% |
| | Bsp. 12p | | |

### Beispiel 11a: Synthese von N-Phenyl-spiro[9H-fluoren-6'-(4,4,5,5-tetramethyl-[1,3,2]dioxaborolan-2-yl)-9,7'(1'H)-indeno[1,2-a]carbazol]

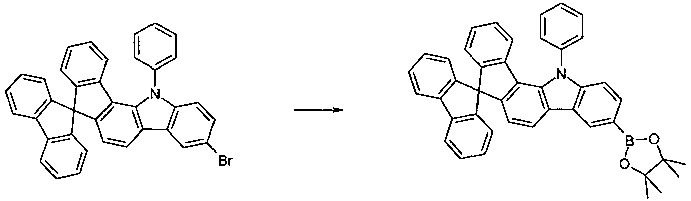

46.0 g (82 mmol) *N*-Phenyl-spiro[9*H*-fluoren-6'-brom-9,7'(1'*H*)-indeno[1,2-*a*]carbazol] (Bsp. 10a), 21.9 g (86 mmol) Bis(pinacolato)diboran, 64.4 g (656 mmol) Kaliumacetat und 2.0 g (2.4 mmol) 1,1'-Bis(diphenyl-phosphino)ferrocen-dichloropalladium(II)-Dichlormethan-Addukt [95464-26-4] werden in 1000 ml Dioxan für 22 h auf 80 °C Innentemperatur erhitzt. Nach Abkühlen auf Raumtemperatur wird das Lösungsmittel am Rotationsverdampfer entfernt, der Rückstand mit 700 ml Dichlormethan und 1000 ml Wasser versetzt und für 30 Minuten gerührt. Die organische Phase wird abgetrennt, zweimal mit je 250 ml Wasser gewaschen, über Natriumsulfat getrocknet und auf etwa 100 ml eingeengt. 1000 ml Heptan werden eingerührt und der gebildete Feststoff abgesaugt. Nach Trocknen im Vakuum verbleiben 43.7 g (72 mmol, 88% der Theorie) des Produkts als hellbrauner Feststoff mit einer Reinheit von etwa 96% nach ¹H-NMR.

In analoger Weise können folgende Verbindungen hergestellt werden:

| Bsp. | Edukt | Produkt | Ausbeute |
|---|---|---|---|
| 11b | 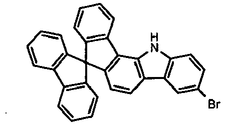 | 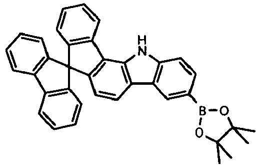 | 81% |
| | Bsp. 10b | | |
| 11c | 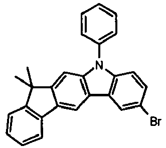 | 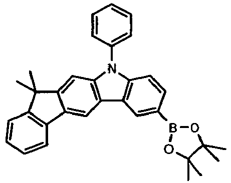 | 89% |
| | Bsp. 10d | | |
| 11d | 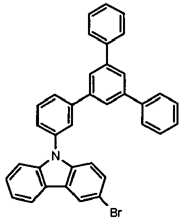 | 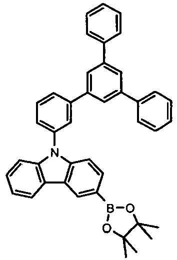 | 82% |
| | Bsp. 10e | | |
| 11e | 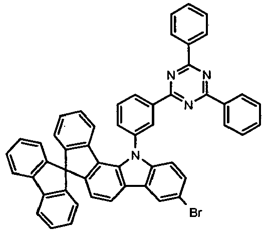 | 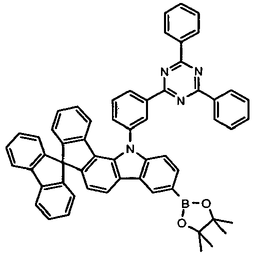 | 78% |
| | Bsp. 10f | | |
| 11f | 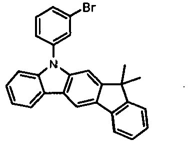 | 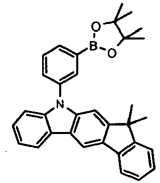 | 63% |
| | Bsp. 4a | | |
| 11g | 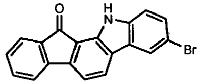 | 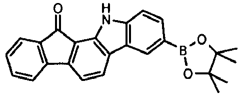 | 61% |
| | Bsp. 10k | | |

### Beispiel 12a: Synthese von Spiro[9H-fluoren-6'-(N-phenylcarbazol-3-yl)-9,7'(1'H)-indeno[1,2-a]carbazol]

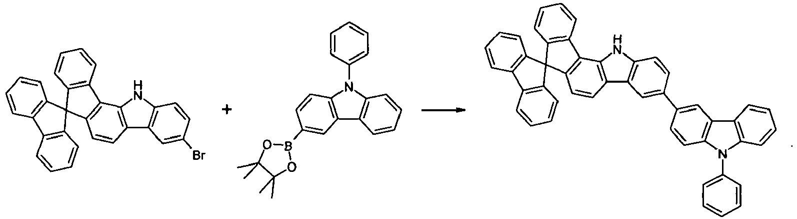

31.0 g (64 mmol) Spiro[9*H*-fluoren-6'-brom-9,7'(1'*H*)-indeno[1,2-*a*]carbazol] (aus Bsp. 10b), 28.4 g (76 mmol) *N*-Phenyl-3-(4,4,5,5-tetramethyl-[1,3,2]di-oxaborolan-2-yl)-carbazol [1126522-69-7] werden in einer Mischung aus 250 ml Toluol, 125 ml Dioxan und 60 ml Wasser vorgelegt und für 30 Minuten mit Argon durchspült. Nach Zugabe von 32.4 g (141 mmol) Trikaliumphosphat-Monohydrat [27176-10-9], 359 mg (1.6 mmol) Palladium-(II)acetat und 974 mg (3.2 mmol) Tri-o-tolylphosphin wird das Reaktionsgemisch für 18 h unter Rückfluss erhitzt. Nach Abkühlen auf Raumtemperatur wird mit 500 ml Wasser erweitert. Die organische Phase wird abgetrennt, zweimal mit je 250 ml Wasser gewaschen und am Rotationsverdampfer vom Lösungsmittel befreit. Der Rückstand wird mit 150 ml Heptan aufgeschlämmt und der gebildete Feststoff abgesaugt. Dieser wird mit etwa 250 ml Cyclohexan über Aluminiumoxid (basisch, Aktivitätsstufe 1) heißextrahiert; gegebenenfalls wird die Extraktionslösung um etwa ein Drittel reduziert. Der gebildete Feststoff wird abgesaugt, und nach Trocknen im Vakuum verbleiben 21.7 g (34 mmol, 53% der Theorie) des Produkts als beigefarbener Feststoff mit einer Reinheit von etwa 99% nach ¹H-NMR.

In analoger Weise können folgende Verbindungen hergestellt werden:

| Bsp. | Edukt | Produkt | Ausbeute |
|---|---|---|---|
| 12b | 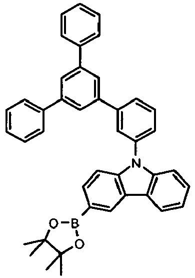 | 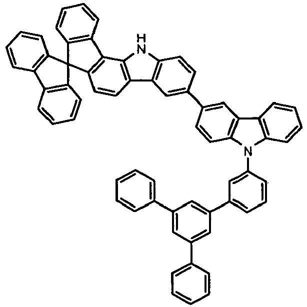 | 56% |
| | Bsp. 11d | | |
| 12c | 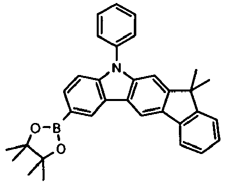 | 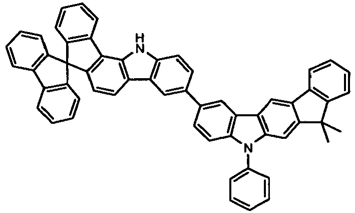 | 55% |
| | Bsp. 11c | | |
| 12d | 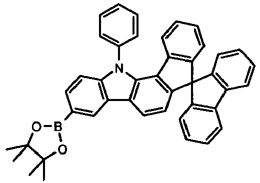 | 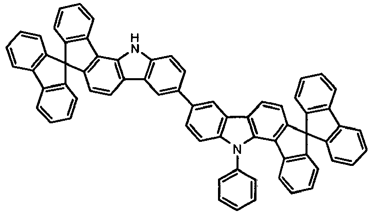 | 32% |
| | Bsp. 11a | | |
| 12e | 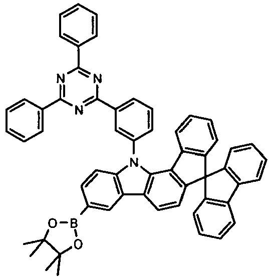 | 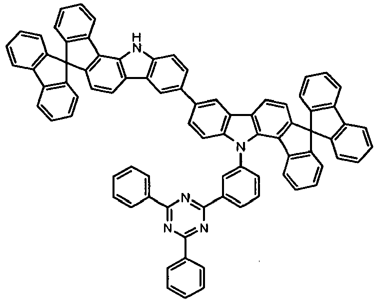 | 47% |
| | Bsp. 11e | | |
| 12f | 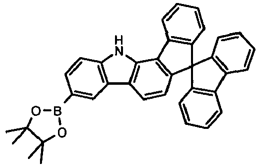 | 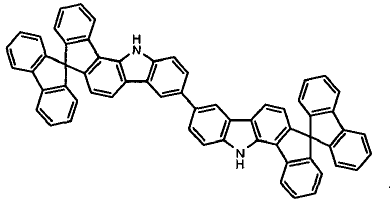 | 36% |
| | Bsp. 11b | | |
| 12g | 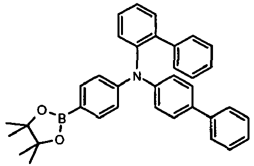 | 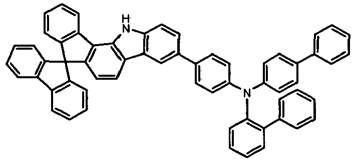 | 75% |
| | Bsp. 2a | | |

In analoger Weise können durch Umsetzung entsprechender Bromide mit 12,12-Dimethyl-10-phenyl-7-(4,4,5,5-tetramethyl-[1,3,2]dioxaborolan-2-yl)-10,12-dihydro-10-aza-indeno[2,1-b]fluoren (Bsp. 11c) folgende Verbindungen hergestellt werden:

| Bsp. | Edukt | Produkt | Ausbeute |
|---|---|---|---|
| 12h | 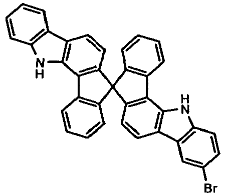 | 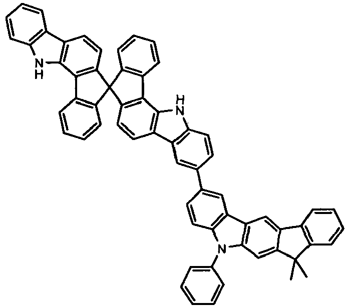 | 50% |
| | Bsp. 10g | | |
| 12i | 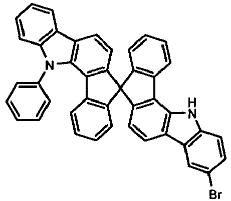 | 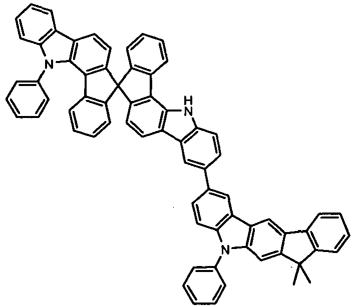 | 52% |
| | Bsp. 10i | | |
| 12j | 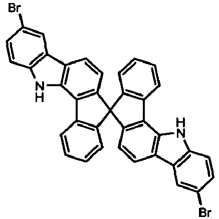 | 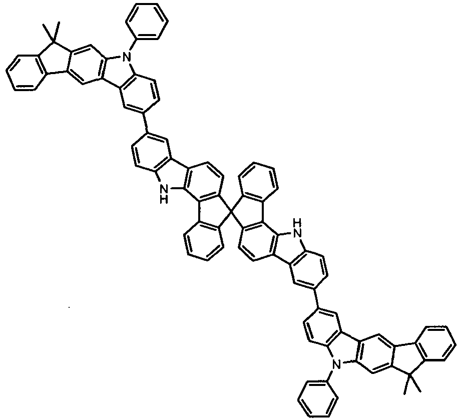 | 28% |
| | Bsp. 10h | | |

In analoger Weise können durch Umsetzung entsprechender Bromide mit *N*-Phenyl-3-(4,4,5,5-tetramethyl-[1,3,2]dioxaborolan-2-yl)-carbazol [1126522-69-7] folgende Verbindungen hergestellt werden:

| Bsp. | Edukt | Produkt | Ausbeute |
|---|---|---|---|
| 12k | 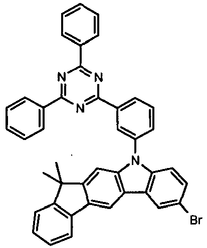 | 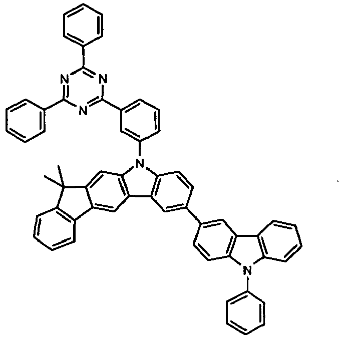 | 69% |
| | Bsp. 10j | | |
| 12l | 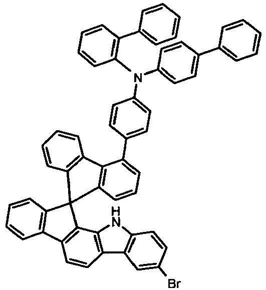 | 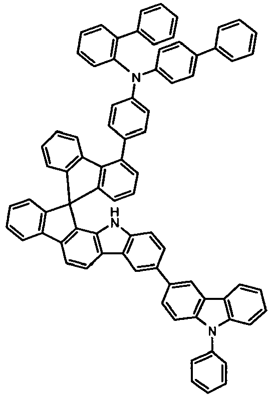 | 55% |
| | Bsp. 10l | | |

In analoger Weise können folgende Verbindungen hergestellt werden:

| Bsp. | Edukt 1 | Edukt 2 | Produkt | Ausbeute |
|---|---|---|---|---|
| 12m | 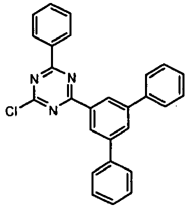 | 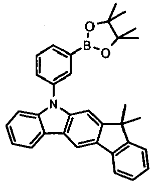 | 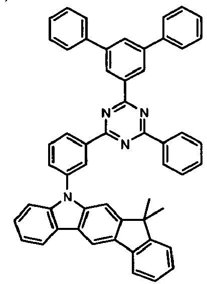 | 36% |
| | Bsp. 3c | Bsp. 11f | | |
| 12n | 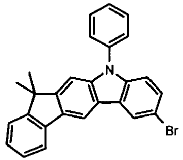 | 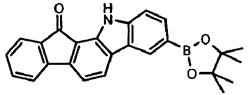 | 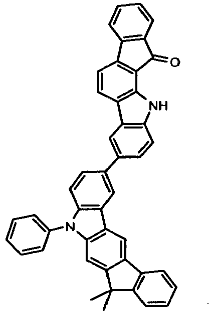 | 41% |
| | Bsp. 10d | Bsp. 11g | | |
| 12o | 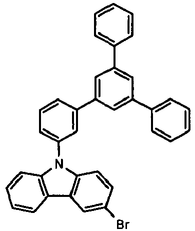 | 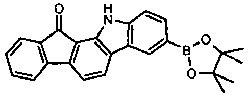 | 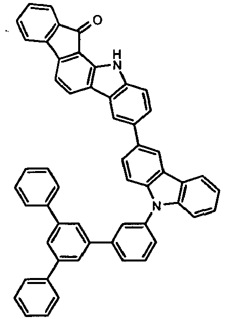 | 44% |
| | Bsp. 10e | Bsp. 11g | | |
| 12p | 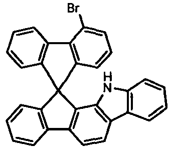 | 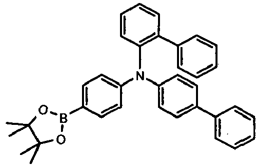 | 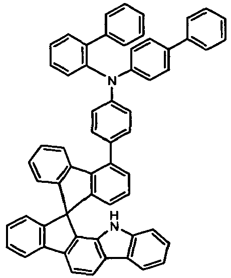 | 15% nach Säulenchroma -tographie |
| | Bsp. 9c | Bsp. 2a | | |

### Beispiel 13a: Synthese von 12'-[3-(4,6-diphenyl-1,3,5-triazin-2-yl)phenyl]-3'-(9-phenylcarbazol-3-yl)spiro[fluoren-9,7'-indeno[1,2-a]-carbazol]

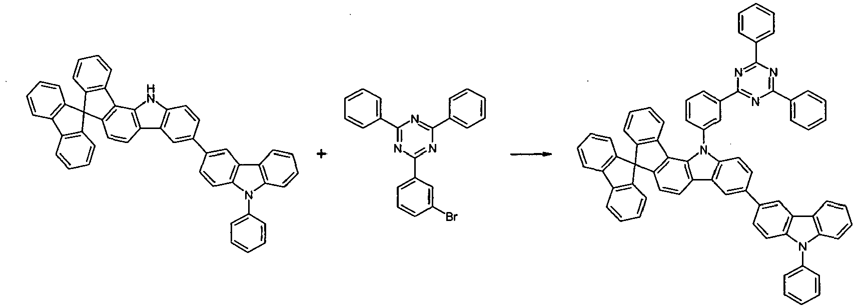

21.7 g (34 mmol) 3-(9-phenylcarbazol-3-yl)spiro[12*H*-indeno[1,2-*a*]-carbazol-7,9'-fluoren] (aus Bsp. 12a) und 13.2 g (34 mmol) 2-(3-Bromphenyl)-4,6-diphenyl-[1,3,5]triazin [1233200-57-1] werden in 280 ml Toluol vorgelegt und 30 Minuten mit Argon durchspült. Nach Zugabe von 175 mg (0.3 mmol) Bis(dibenzylidenaceton)palladium(0), 250 mg (0.6 mmol) Dicyclohexyl-(2',6'-dimethoxy-biphenyl-2-yl)-phosphin und 4.4 g (46 mmol) Natrium-*tert-*butylat wird das Reaktionsgemisch für 45 h unter Rückfluss erhitzt. Nach Abkühlen auf Raumtemperatur werden 350 ml Wasser eingerührt, die organische Phase abgetrennt und vom Lösungmittel befreit. Der Rückstand wird in Toluol aufgenommen; dann wird das dreifache Volumen Heptan eingerührt, der gebildete Feststoff abgesaugt und einmal aus Toluol umkristallisiert. Abschließend wird das Produkt säulenchromatographisch über Kieselgel mit einem Toluol/Heptan-Laufmittelgemisch (2:1) gereinigt. Nach Trocknen im Vakuum verbleiben 10.8 g (12 mmol, 36% der Theorie) des Produkts als schwach gelber Feststoff mit einer Reinheit von 99.8% nach HPLC.

In analoger Weise kann auch folgende Verbindung hergestellt werden:

| Bsp. | Edukt | Produkt | Ausbeute |
|---|---|---|---|
| 13b | 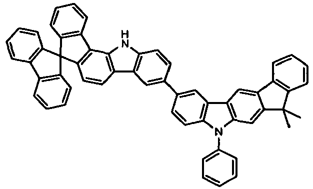 | 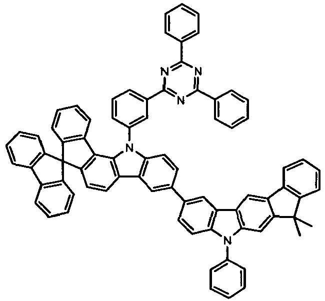 | 39% |
| | Bsp. 12c | | |

### Beispiel 14a: Synthese von 12'-(4,6-diphenyl-1,3,5-triazin-2-yl)-3'-(9-phenylcarbazol-3-yl)spiro[fluoren-9,7'-indeno[1,2-a]carbazol]

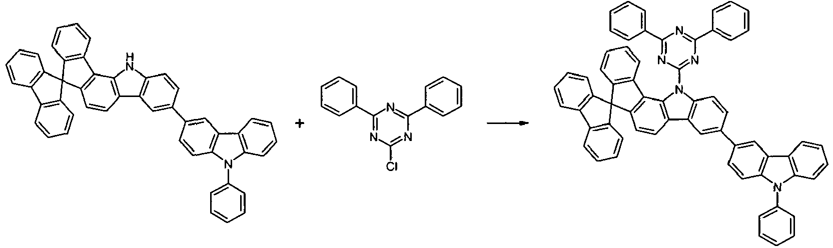

Eine Lösung von 67.9 g (105 mmol) 3-(9-phenylcarbazol-3-yl)spiro[12*H-*indeno[1,2-*a*]carbazol-7,9'-fluoren] (Bsp. 12a) in 300 ml Lösungsmittel Dimethylformanid wird unter kräftigem Rühren in eine Lösung von 4.2 g Natriumhydrid (60% in Mineralöl, 105 mmol) in 300 ml Dimethylformamid getropft und 2 h gerührt. Dann wird eine Lösung von 30.0 g (112 mmol) 2-Chlor-4,6-diphenyl-[1,3,5]triazin [3842-55-5] in 200 ml THF langsam zugetropft, das Gemisch für 18 h gerührt und anschließend auf etwa 150 g Eis gegossen. Die organische Phase wird mit 250 ml Toluol erweitert, abgetrennt, zweimal mit 100 ml Wasser gewaschen und am Rotationsverdampfer vom Lösungsmittel befreit. Der verbliebene Rückstand wird mit Toluol über Aluminiumoxid (basisch, Aktivitätsstufe 1) heißextrahiert; die gebildete Suspension wird am Rotationsverdampfer vom Lösungsmittel befreit und der verbliebene Rückstand säulenchromatographisch über Kieselgel mit einem Heptan/ THF-Laufmittelgemisch (4:1) gereinigt. Nach Entfernen der Lösungsmittel am Rotationsverdampfer und Tempern des Rückstandes bei 180 °C und einem Druck von etwa 10⁻⁵ mbar für 5 h verbleiben 31.5 g (33 mmol, 31% der Theorie) des Produkts als farbloser Feststoff mit einer Reinheit von 99.9% nach HPLC.

In analoger Weise können folgende Verbindungen hergestellt werden:

| Bsp. | Edukt 1 | Edukt 2 | Produkt | Ausbeute |
|---|---|---|---|---|
| 14b | 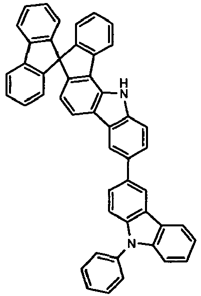 | 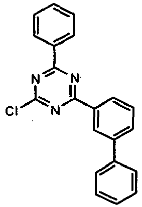 | 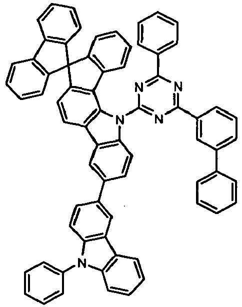 | 28% |
| | Bsp. 12a | Bsp. 3a | | |
| 14c | 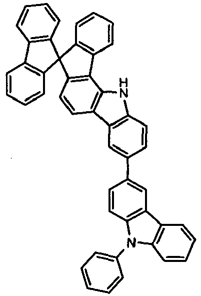 | 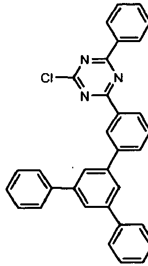 | 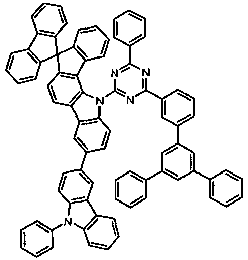 | 37% |
| | Bsp. 12a | Bsp. 3b | | |
| 14d | 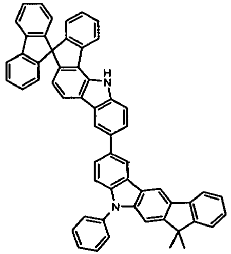 | 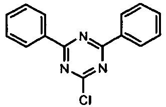 | 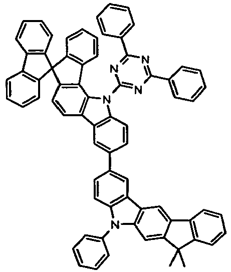 | 36% |
| | Bsp. 12c | [3842-55-5] | | |
| 14e | 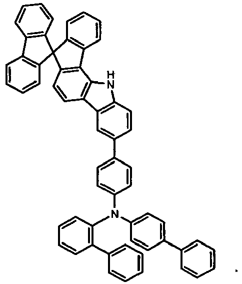 | 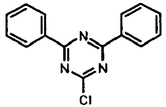 | 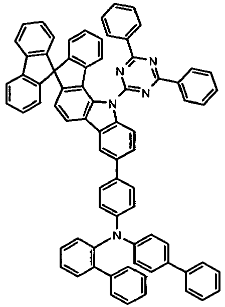 | 40% |
| | Bsp. 12g | [3842-55-5] | | |
| 14f | 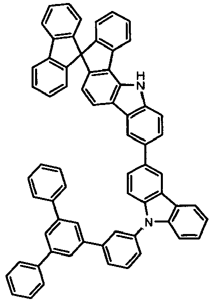 | 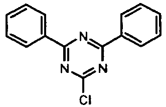 | 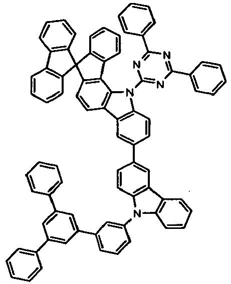 | 31% |
| | Bsp. 12b | [3842-55-5] | | |
| 14g | 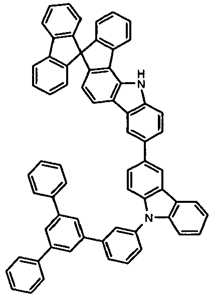 | 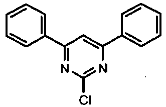 | 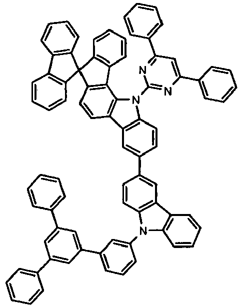 | 35% |
| | Bsp. 12b | [2915-16-4] | | |
| 14h | 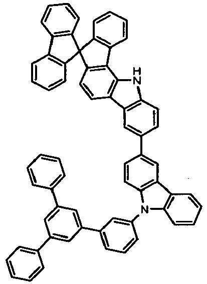 | 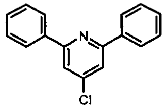 | 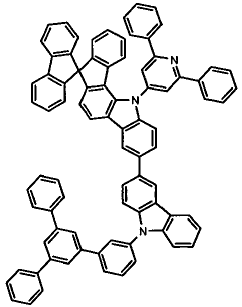 | 26% |
| | Bsp. 12b | [78500-89-7] | | |
| 14i | 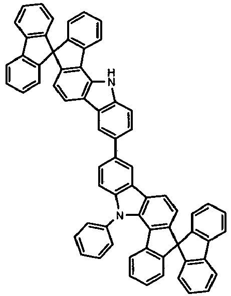 | 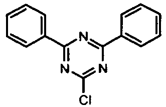 | 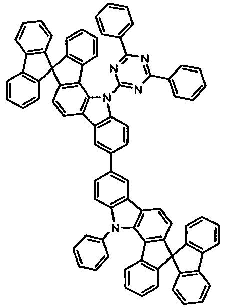 | 30% |
| | Bsp. 12d | [3842-55-5] | | |
| 14j | 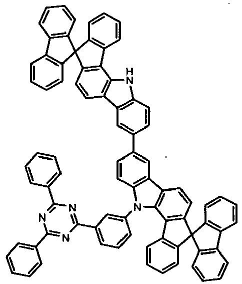 | 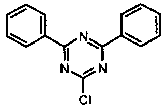 | 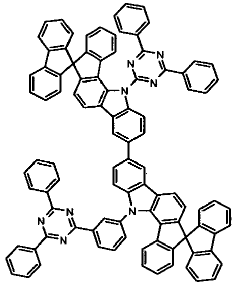 | 27% |
| | Bsp. 12e | [3842-55-5] | | |
| 14k | 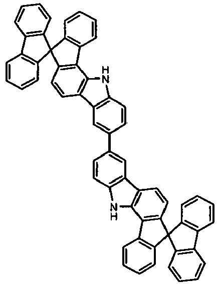 | 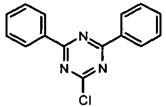 | 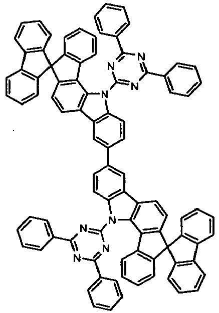 | 41% |
| | Bsp. 12f | [3842-55-5] | | |
| 14l | 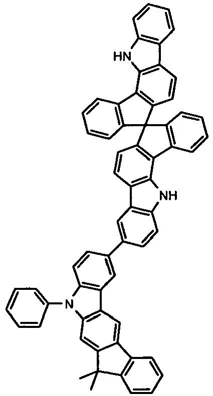 | 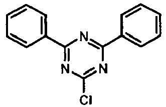 | 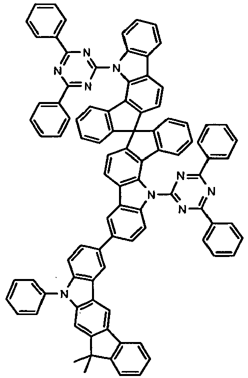 | 33% |
| | Bsp. 12h | [3842-55-5] | | |
| 14m | 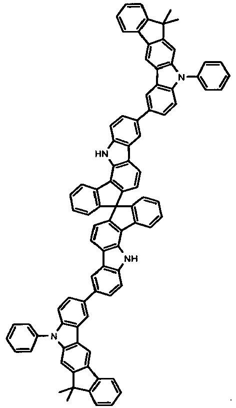 | 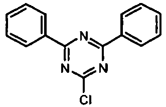 | 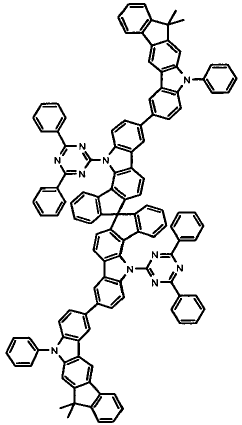 | 36% |
| | Bsp. 12j | [3842-55-5] | | |
| 14n | 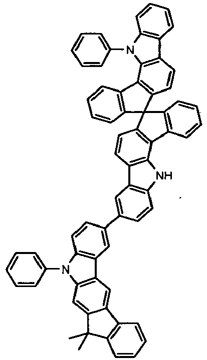 | 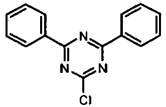 | 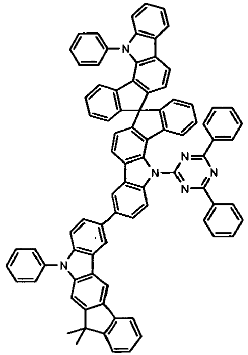 | 43% |
| | Bsp. 12i | [3842-55-5] | | |
| 14o | 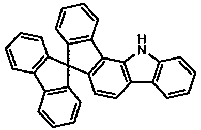 | 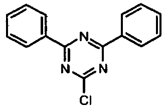 | 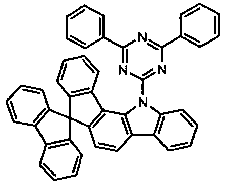 | 45% |
| | Bsp. 7a | [3842-55-5] | | |
| 14p | 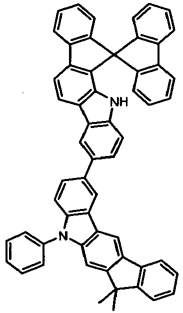 | 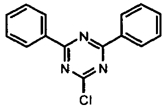 | 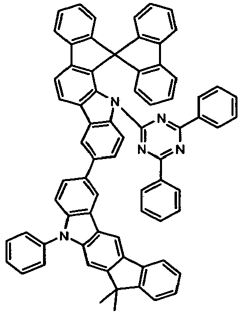 | 38% |
| | Bsp. 9d | [3842-55-5] | | |
| 14q | 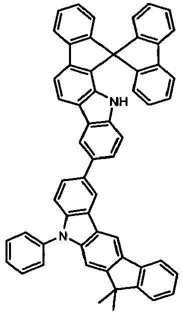 | 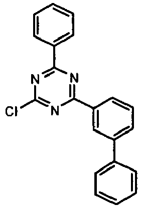 | 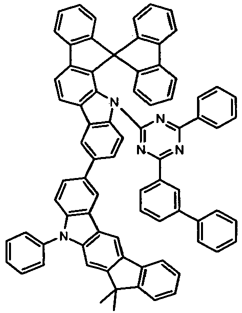 | 39% |
| | Bsp. 9d | Bsp. 3a | | |
| 14r | 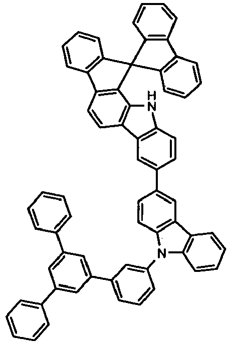 | 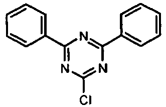 | 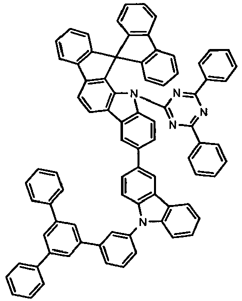 | 28% |
| | Bsp.9e | [3842-55-5] | | |
| 14s | 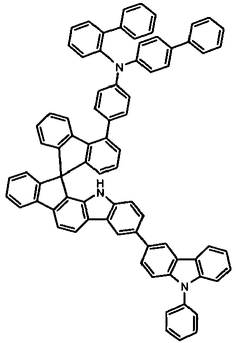 | 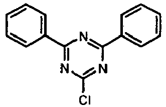 | 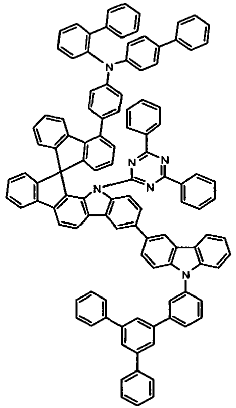 | 17% |
| | Bsp. 12l | [3842-55-5] | | |

### Beispiele AV2 und AV4

Die Vergleichsbeispiele AV2 und AV4 können nach den in DE 102008017591 beschriebenen Verfahren hergestellt werden.

### Beispiel AV6

Das Vergleichsbeispiel AV6 kann nach den in WO 2011/132683 beschriebenen Verfahren hergestellt werden.

### Devicebeispiele:

### Devicebeispiel 1: Herstellung lösungsprozessierter OLEDs

Die erfindungsgemäßen Materialien können aus Lösung verarbeitet werden und führen gegenüber vakuumprozessierten OLEDs zu wesentlich einfacher herstellbaren OLEDs mit dennoch guten Eigenschaften. Zum Beispiel können die Vergleichsmaterialien 12m und 14o (Tabelle 2) nicht in einer Konzentration von 15 mg/ml in Toluol gelöst werden, während dies mit den erfindungsgemäßen Materialien 14a, 14g, 13b und 14e problemlos möglich ist (Tabelle 1).

Um zu überprüfen, ob bei einem Material eine Löslichkeit von 15 mg/ml oder mehr in Toluol gegeben ist, wird wie folgt verfahren: 30 mg des Materials werden als Feststoff in einem Probenfläschchen vorgelegt. Bei Raumtemperatur werden 2 ml Toluol zugegeben. Das Fläschchen wird verschlossen und der Inhalt auf einem beheizbaren Magnetrührer 1 h bei 60 °C gerührt. Dabei wird mittels eines Aluminiumblocks mit Löchern, in die die Fläschchen genau hineinpassen, für guten thermischen Kontakt gesorgt. Nach 1 h wird das Fläschchen entnommen und auf Raumtemperatur abkühlen lassen. Befindet sich dann eine klare Lösung ohne größere Partikel im Fläschchen, so sind mindestens 15 mg/ml des Materials in Toluol löslich.

Des Weiteren erlaubt die höhere Glasübergangstemperatur der erfindungsgemäßen Matrixmaterialien das Ausheizen bei höheren Temperaturen und bietet damit ein größeres Prozessierungsfenster als die Vergleichsmaterialien 12m und 14o.

**Tabelle 1: Für die Prozessierbarkeit aus Lösung relevante Eigenschaften ausgewählter Matrixmaterialien**

| Verbindung aus Bsp. | Löslichkeit in Toluol > 15 mg/ml | Tg > 175°C |
|---|---|---|
| 12m | nein | nein |
| 14o | nein | nein |
| 14a | ja | ja |
| 14g | ja | ja |
| 13b | ja | ja |
| 14e | ja | ja |

Die Herstellung vollständig lösungsbasierter OLEDs ist in der Literatur bereits vielfach beschrieben, z. B. in WO 2004/037887. Die Herstellung vakuumbasierter OLEDs ist ebenfalls vielfach vorbeschrieben, u.a. in WO 2004/058911. In den im Folgenden diskutierten Beispielen werden lösungsbasiert und vakuumbasiert aufgebrachte Schichten innerhalb einer OLED kombiniert, so dass die Prozessierung bis einschließlich zur Emissionsschicht aus Lösung und in den darauffolgenden Schichten (Lochblockierschicht und Elektronentransportschicht) aus dem Vakuum erfolgte. Die vorbeschriebenen allgemeinen Verfahren werden dafür wie folgt auf die hier beschriebenen Gegebenheiten (Schichtdickenvariation, Materialien) angepasst und kombiniert:
Der Aufbau ist wie folgt:
- Substrat,
- ITO (50 nm)
- PEDOT:PSS (20 bzw. 60nm für grüne bzw. rote Bauteile)
- Lochtransportschicht (HTL) (20 nm)
- Emissionsschicht (EML) (60 nm)
- Lochblockierschicht (HBL) (10 nm)
- Elektronentransportschicht (ETL) (40 nm)
- Kathode.

Als Substrat dienen Glasplättchen, die mit strukturiertem ITO (Indium Zinn Oxid) der Dicke 50 nm beschichtet sind. Zur besseren Prozessierung werden diese mit PEDOT:PSS beschichtet (Poly(3,4-ethylendioxy-2,5-thiophen) : Polystyrolsulfonat, bezogen von Heraeus Precious Metals GmbH & Co. KG, Deutschland). PEDOT:PSS wird an Luft aus Wasser aufgeschleudert und nachfolgend an Luft bei 180°C für 10 Minuten ausgeheizt, um Restwasser zu entfernen. Auf diese beschichteten Glasplättchen werden die Interlayer sowie die Emissionsschicht aufgebracht. Die verwendete Lochtransportschicht ist vernetzbar. Es wird ein Polymer der nachfolgend gezeigten Struktur verwendet, das gemäß WO2010/097155 synthetisiert werden kann.

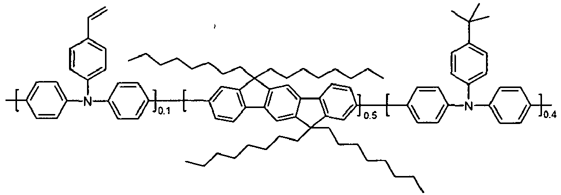

Das Lochtransport-Polymer wird in Toluol gelöst. Der typische Feststoffgehalt solcher Lösungen liegt bei ca. 5 g/l, wenn, wie hier, die für eine Device typische Schichtdicke von 20 nm mittels Spincoating erzielt werden soll. Die Schichten werden in einer Inertgasatmosphäre, im vorliegenden Fall Argon, aufgeschleudert und 60 Minuten bei 180 °C ausgeheizt.

Die Emissionsschicht setzt sich immer aus mindestens einem Matrixmaterial (Hostmaterial, Wirtsmaterial) und einem emittierenden Dotierstoff (Dotand, Emitter) zusammen. Weiterhin auftreten können Mischungen aus mehreren Matrixmaterialien sowie Co-Dotanden. Eine Angabe wie TMM-A (92%) : Dotand (8%) bedeutet hierbei, dass das Material TMM-A in einem Gewichtsanteil von 92% und Dotand in einem Gewichtsanteil von 8% in der Emissionsschicht vorliegt. Die Mischung für die Emissionsschicht wird in Toluol oder ggf. Chlorbenzol gelöst. Der typische Feststoffgehalt solcher Lösungen liegt bei ca. 18 g/l, wenn, wie hier, die für eine Device typische Schichtdicke von 60 nm mittels Spincoating erzielt werden soll. Die Schichten werden in einer Inertgasatmosphäre, im vorliegenden Fall Argon, aufgeschleudert und 10 Minuten bei 160 °C ausgeheizt. Verwendete Matrixmaterialien sind in Tabelle 2 aufgeführt - es handelt sich hierbei sowohl um erfindungsgemäße Verbindungen als auch um Vergleichsbeispiele.

**Tabelle 2: Strukturen erfindungsgemäßer Matrixmaterialien sowie Vergleichsmatrices**

| | |
|---|---|
| 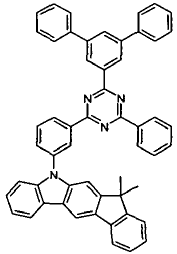 | 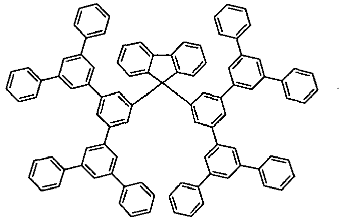 |
| 12m (SdT) | AV2 |
| 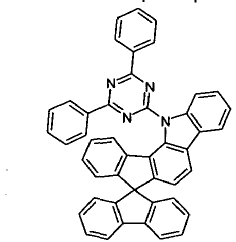 | 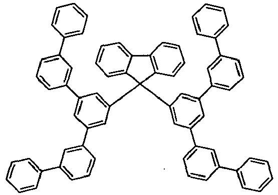 |
| 14o | AV4 |
| 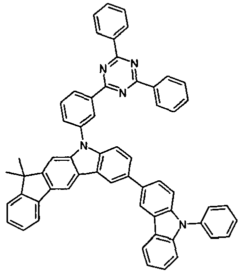 | 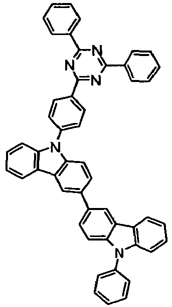 |
| 12k | AV6 |
| 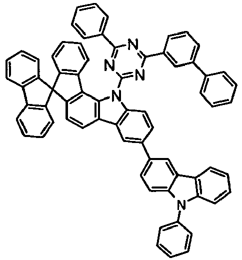 | 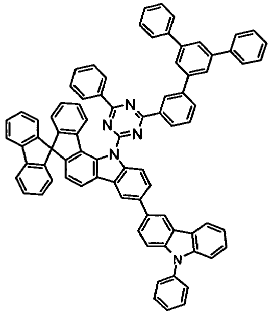 |
| 14b | 14c |
| 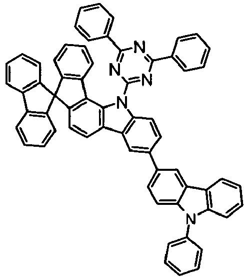 | 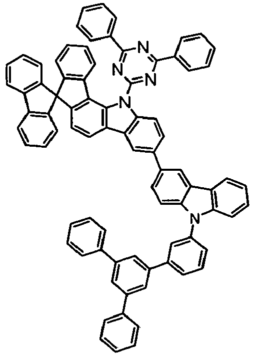 |
| 14a | 14f |
| 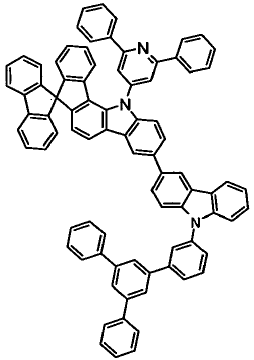 | 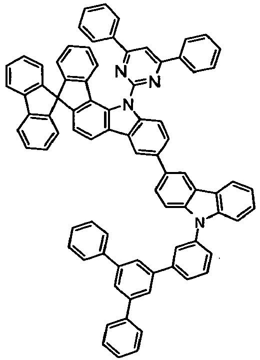 |
| 14h | 14g |
| 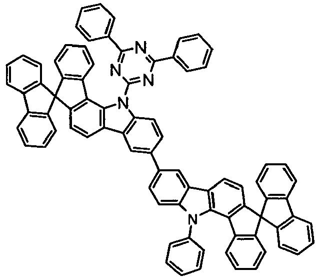 | 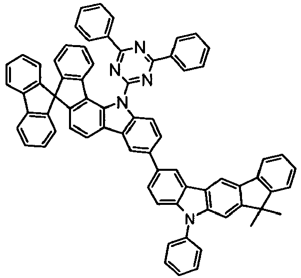 |
| 14i | 14d |
| 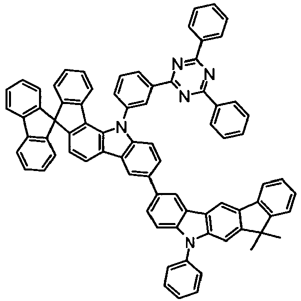 | 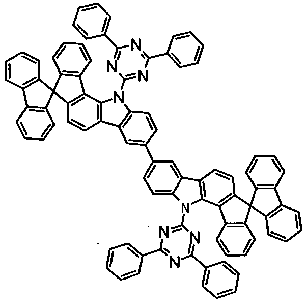 |
| 13b | 14k |
| 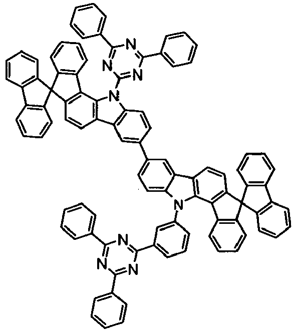 | |
| 14j | |
| 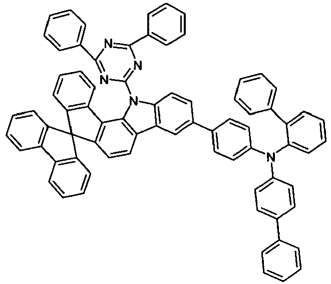 | 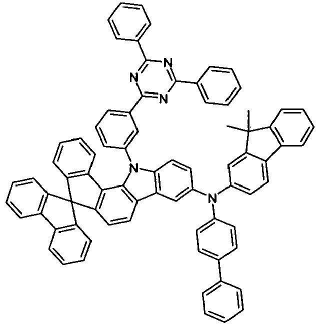 |
| 14e | 8d |
| 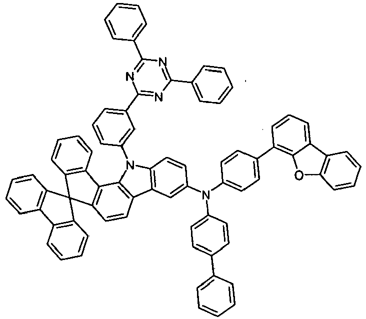 | 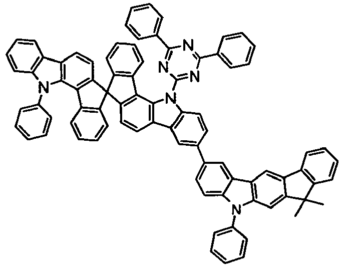 |
| 8e | 14n |
| 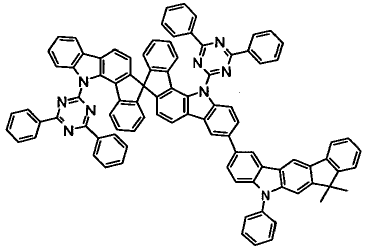 | 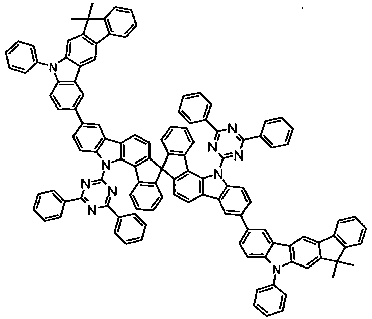 |
| 14l | 14m |
| 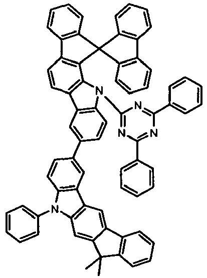 | 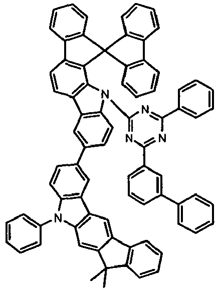 |
| 14p | 14q |
| 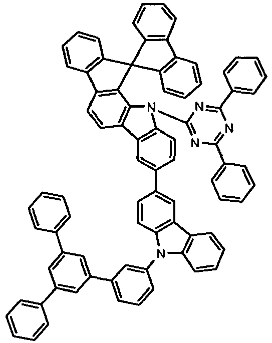 | 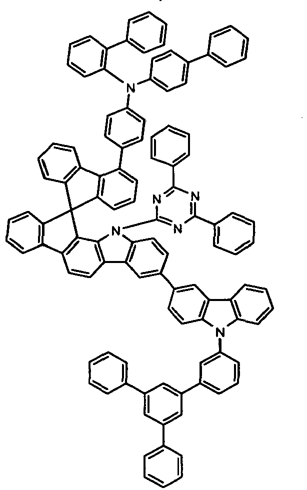 |
| 14r | 14s |

Die im vorliegenden Fall verwendeten Dotanden sind in Tabelle 3 gezeigt.

**Tabelle 3: Verwendete Dotanden**

| | | |
|---|---|---|
| 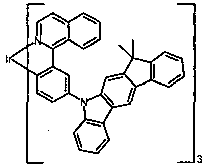 | 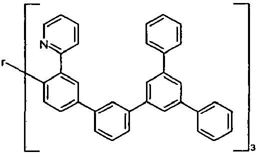 | 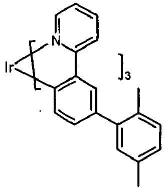 |
| D1 | D2 | D3 |

Die Materialien für die Lochblockierschicht und Elektronentransportschicht werden in einer Vakuumkammer thermisch aufgedampft. Dabei kann z. B. die Elektronentransportschicht aus mehr als einem Material bestehen, die einander durch Co-Verdampfung in einem bestimmten Volumenanteil beigemischt werden. Eine Angabe wie ETM1:ETM2 (50%:50%) bedeutet hierbei, dass die Materialien ETM1 und ETM2 in einem Volumenanteil von je 50% in der Schicht vorliegen. Die im vorliegenden Fall verwendeten Materialien sind in Tabelle 4 gezeigt.

**Tabelle 4: Verwendete HBL- und ETL-Materialien**

| | |
|---|---|
| 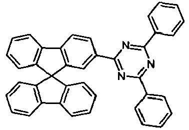 | 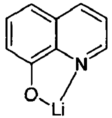 |
| ETM1 | ETM2 |

Die Kathode wird durch die thermische Aufdampfung einer 100 nm dicken Aluminiumschicht gebildet. Die OLEDs werden standardmäßig charakterisiert. Hierfür werden die Elektrolumineszenzspektren, Strom-Spannungs-Leuchtdichte-Kennlinien (IUL-Kennlinien) unter Annahme einer Lambert'schen Abstrahlcharakteristik sowie die (Betriebs-)Lebensdauer bestimmt. Aus den IUL-Kennlinien werden Kennzahlen bestimmt wie die Betriebsspannung U (in V) und die externe Quanteneffizienz (in %) bei einer bestimmten Helligkeit. LD80 @ 8000 cd/m² ist die Lebensdauer, bis die OLED bei einer Starthelligkeit von 8000 cd/m² auf 80 % der Anfangsintensität, also auf 6400 cd/m², abgefallen ist. Entsprechend ist LD80 @ 10000 cd/m² die Lebensdauer, bis die OLED bei einer Starthelligkeit von 10000 cd/m² auf 80 % der Anfangsintensität, also auf 8000 cd/m², abgefallen ist.

Die Daten von OLEDs, deren EML aus TMM-A, TMM-B und Dotand D (gemäß Tabelle 2 und Tabelle 3) bestehen, sind in Tabelle 5 gezeigt. Dabei wurde ETM-1 als HBL und ETM1:ETM2 (50%:50%) als ETL verwendet.

**Tabelle 5: Ergebnisse lösungsprozessierter OLEDs mit EML-Mischungen vom Typ x% TMM-A, (100-x-y)% TMM-B, y % Dotand D**

| Bsp. | TMM-A | | TMM-B | | Dotand D | | Effizienz bei 1000 cd/m² | Spannung bei 1000 cd/m² | LD80 bei 8000 cd/m² |
|---|---|---|---|---|---|---|---|---|---|
| | Material | % | Material | % | Material | % | % EQE | [V] | [h] |
| V1 | 12m | 40 | AV2 | 30 | D2 | 30 | 19.0 | 5.5 | 236 |
| 1 | 14b | 40 | AV2 | 30 | D2 | 30 | 19.8 | 5.2 | 289 |
| V2 | 12m | 40 | AV2 | 40 | D2 | 20 | 16.0 | 5.2 | 166 |
| V6 | AV6 | 40 | AV2 | 40 | D2 | 20 | 18.4 | 5.1 | 267 |
| 2 | 14b | 40 | AV2 | 40 | D2 | 20 | 19.4 | 5.1 | 417 |
| 3 | 14c | 40 | AV2 | 40 | D2 | 20 | 19.5 | 4.9 | 410 |
| 4 | 14a | 40 | AV2 | 40 | D2 | 20 | 18.9 | 5.1 | 380 |
| 5 | 14f | 40 | AV2 | 40 | D2 | 20 | 19.5 | 5.0 | 337 |
| 6 | 14h | 40 | AV2 | 40 | D2 | 20 | 17.2 | 5.5 | 293 |
| 7 | 14g | 40 | AV2 | 40 | D2 | 20 | 19.7 | 5.3 | 406 |
| 8 | 14i | 40 | AV2 | 40 | D2 | 20 | 19.5 | 5.0 | 392 |
| 9 | 14d | 40 | AV2 | 40 | D2 | 20 | 19.4 | 5.1 | 364 |
| 10 | 13b | 40 | AV2 | 40 | D2 | 20 | 19.3 | 5.1 | 374 |
| 11 | 14k | 40 | AV2 | 40 | D2 | 20 | 18.4 | 5.4 | 359 |
| 12 | 14j | 40 | AV2 | 40 | D2 | 20 | 18.6 | 5.5 | 344 |
| 13 | 14e | 40 | AV2 | 40 | D2 | 20 | 19.0 | 5.3 | 317 |
| 14 | 8d | 40 | AV2 | 40 | D2 | 20 | 18.8 | 5.1 | 331 |
| 15 | 8e | 40 | AV2 | 40 | D2 | 20 | 18.5 | 5.0 | 332 |
| 16 | 14n | 40 | AV2 | 40 | D2 | 20 | 19.5 | 5.1 | 367 |
| 17 | 14l | 40 | AV2 | 40 | D2 | 20 | 19.4 | 5.2 | 350 |
| 18 | 14m | 40 | AV2 | 40 | D2 | 20 | 19.4 | 5.1 | 364 |
| 19 | 14p | 40 | AV2 | 40 | D2 | 20 | 18.8 | 5.2 | 308 |
| 20 | 14q | 40 | AV2 | 40 | D2 | 20 | 19.2 | 5.2 | 324 |
| 21 | 14r | 40 | AV2 | 40 | D2 | 20 | 19.0 | 5.1 | 341 |
| 22 | 14s | 40 | AV2 | 40 | D2 | 20 | 19.4 | 5.0 | 288 |
| V3 | 12m | 20 | AV2 | 60 | D2 | 20 | 18.6 | 5.3 | 240 |
| 23 | 14c | 20 | AV2 | 60 | D2 | 20 | 20.1 | 5.2 | 551 |

Die Daten von OLEDs, deren EML aus TMM-A, TMM-B, Co-Dotand D1 und Dotand D (gemäß Tabelle 2 und Tabelle 3) bestehen, sind in Tabelle 6 gezeigt. Dabei wird ETM-1 als HBL und ETM1:ETM2 (50%:50%) als ETL verwendet.

**Tabelle 6: Ergebnisse lösungsprozessierter OLEDs mit EML-Mischungen vom Typ x% TMM-A, (100-x-y-z)% TMM-B, z% Co-Dotand C, y % Dotand D**

| Bsp. | TMM-A | | TMM-B | | Co-Dotand C | | Dotand D | | Eff. bei 1000 cd /m² | U bei 1000 cd/m2 | LD80 bei 8000 cd/m² |
|---|---|---|---|---|---|---|---|---|---|---|---|
| | Material | % | Material | % | Material | % | Material | % | % EQE | [V] | [h] |
| V4 | 12m | 39 | AV2 | 45 | D1 | 10 | D3 | 6 | 14.1 | 7.3 | 75 |
| 24 | 14f | 39 | AV2 | 45 | D1 | 10 | D3 | 6 | 14.2 | 6.9 | 178 |
| V5 | 12m | 40 | AV2 | 24 | D1 | 30 | D3 | 6 | 13.2 | 6.7 | 162 |
| 25 | 14f | 40 | AV2 | 24 | D1 | 30 | D3 | 6 | 13.4 | 6.6 | 244 |

### Devicebeispiel 2: Herstellung vakuumprozessierter OLED

Viele der erfindungsgemäßen Materialien können auch vakuumverdampft werden. In den im Folgenden diskutierten Beispielen werden ausschließlich vakuumbasiert aufgebrachte Schichten verwendet. Die vorbeschriebenen allgemeinen Verfahren werden dafür auf die hier beschriebenen Gegebenheiten (Schichtdickenvariation, Materialien) angepasst. Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs sowie die resultierenden Ergebnisse sind Tabelle 8 zu entnehmen. Die zur Herstellung der OLEDs benötigten Hilfsmaterialien sind in Tabelle 7 gezeigt; erfindungsgemäße Materialien und Vergleichsmaterialien finden sich in Tabelle 2.

**Tabelle 7: Strukturen der verwendeten Hilfsmaterialien**

| | |
|---|---|
| 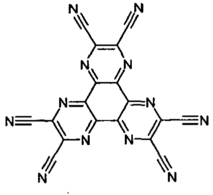 | 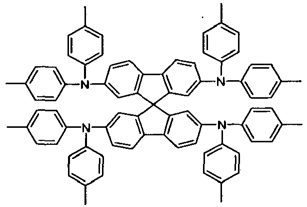 |
| HATCN | SpA1 |
| 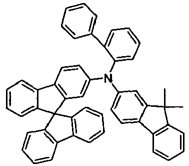 | 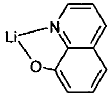 |
| SpMA1 | LiQ |
| 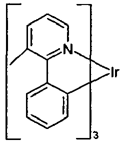 | 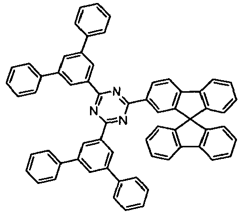 |
| TEG1 | ST2 |
| 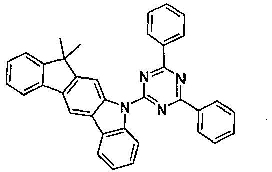 | |
| SDT1 | |

**Tabelle 8: Aufbau vakuumprozessierter OLEDs**

| Bsp. | HTL Dicke | IL Dicke | EBL Dicke | EML Dicke | HBL Dicke | ETL Dicke |
|---|---|---|---|---|---|---|
| V6 | SpA1 70nm | HATCN 5nm | SpMA1 90nm | 12k:TEG1 (90%:10%) 30nm | SDT1 10nm | ST2:LiQ (50%:50%) 30nm |
| 26 | SpA1 70nm | HATCN 5nm | SpMA1 90nm | 14b:TEG1 (90%:10%) 30nm | SDT1 10nm | ST2:LiQ (50%:50%) 30nm |
| 27 | SpA1 70nm | HATCN 5nm | SpMA1 90nm | 12m:14e:TEG1 (58%:30%:12%) 30nm | SDT1 10nm | ST2:LiQ (50%:50%) 30nm |
| 28 | SpA1 70nm | HATCN 5nm | SpMA1 90nm | 14b:AV4:TEG1 (58%:30%:12%) 30nm | SDT1 10nm | ST2:LiQ (50%:50%) 30nm |

**Tabelle 9: Ergebnisse vakuumprozessierter OLEDs**

| Bsp. | Effizienz bei 1000 cd/m 2 | Spannung bei 1000 cd/m² | LD80 bei 20 mA/cm² |
|---|---|---|---|
| V6 | 3.3 | 15.60% | 110 |
| 26 | 3.3 | 15.80% | 145 |
| 27 | 3.0 | 17.90% | 72 |
| 28 | 3.4 | 19.10% | 205 |

## Patentansprüche

1. Verbindung, **dadurch gekennzeichnet, dass** die Verbindung eine Struktur gemäß Formel (1) oder Formel (2) aufweist, 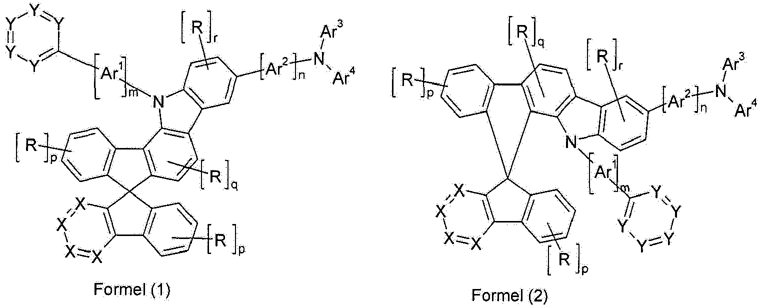 wobei für die verwendeten Symbole und Indizes gilt:
Y ist bei jedem Auftreten gleich oder verschieden CR oder N, mit der Maßgabe, dass mindestens eine Gruppe Y für N steht;
X ist bei jedem Auftreten gleich oder verschieden CR oder N, wobei maximal zwei Gruppen X für N stehen; oder zwei benachbarte X stehen für eine Gruppe der folgenden Formel (3) oder (4) und die weiteren Gruppen X stehen gleich oder verschieden für CR oder N, 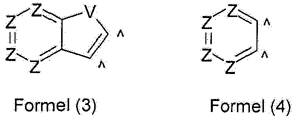 wobei ^ die entsprechenden benachbarten Gruppen X in Formel (1) bzw. Formel (2) andeutet;
V ist bei jedem Auftreten gleich oder verschieden NR, C(R)₂, O, S, BR, Si(R)₂ oder C=O;
Z ist bei jedem Auftreten gleich oder verschieden CR oder N, wobei maximal zwei Gruppen Z für N stehen;
Ar¹ ist ein aromatisches oder heteroaromatisches Ringsystem mit 5 bis 40 aromatischen Ringatomen, das mit einem oder mehreren Resten R substituiert sein kann;
Ar², Ar³, Ar⁴ ist bei jedem Auftreten gleich oder verschieden ein aromatisches oder heteroaromatisches Ringsystem mit 5 bis 40 aromatischen Ringatomen, das mit einem oder mehreren Resten R substituiert sein kann; dabei können Ar² und Ar³ miteinander und/oder Ar³ und Ar⁴ miteinander auch durch eine Einfachbindung oder durch eine Gruppe ausgewählt aus C(R¹)₂, C(R¹)₂-C(R¹)₂, NR¹, O oder S verbunden sein;
R ist bei jedem Auftreten gleich oder verschieden ausgewählt aus der Gruppe bestehend aus H, D, F, Cl, Br, I, CN, NO₂, N(Ar⁵)₂, N(R¹)₂, C(=O)Ar⁵, C(=O)R¹, P(=O)(Ar⁵)₂, P(Ar⁵)₂, B(Ar⁵)₂, Si(Ar⁵)₃, Si(R¹)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann; dabei können optional zwei benachbarte Substituenten R ein monocyclisches oder polycyclisches, aliphatisches, aromatisches oder heteroaromatisches Ringsystem bilden, das mit einem oder mehreren Resten R¹ substituiert sein kann;
Ar⁵ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können zwei Reste Ar⁵, welche an dasselbe N-, P-, B- oder Si-Atom binden, auch durch eine Einfachbindung oder eine Brücke, ausgewählt aus N(R¹), C(R¹)₂, C(R¹)₂-C(R¹)₂, O oder S, miteinander verbrückt sein;
R¹ ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können, wobei zwei oder mehr benachbarte Substituenten R¹ miteinander ein mono- oder polycyclisches, aliphatisches Ringsystem bilden können;
m ist 0 oder 1;
n ist 0 oder 1;
p ist bei jedem Auftreten gleich oder verschieden 0, 1, 2, 3 oder 4;
q ist 0, 1 oder 2;
r ist 0, 1, 2 oder 3;
dabei ist die folgende Verbindung von der Erfindung ausgenommen:

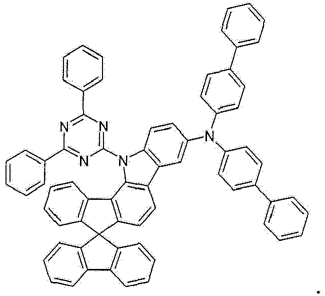

2. Verbindung nach Anspruch 1, **dadurch gekennzeichnet, dass** X gleich oder verschieden bei jedem Auftreten für CR oder N steht, wobei maximal eine Gruppe X pro Cyclus für N steht; oder zwei benachbarte Gruppen X stehen für eine Gruppe der Formel (3), wobei Z gleich oder verschieden bei jedem Auftreten für CR steht und V für NR, C(R)₂, O oder S steht, und die weiteren X stehen für CR.

3. Verbindung nach Anspruch 1 oder 2, ausgewählt aus den Verbindungen der Formeln (5) bis (10), 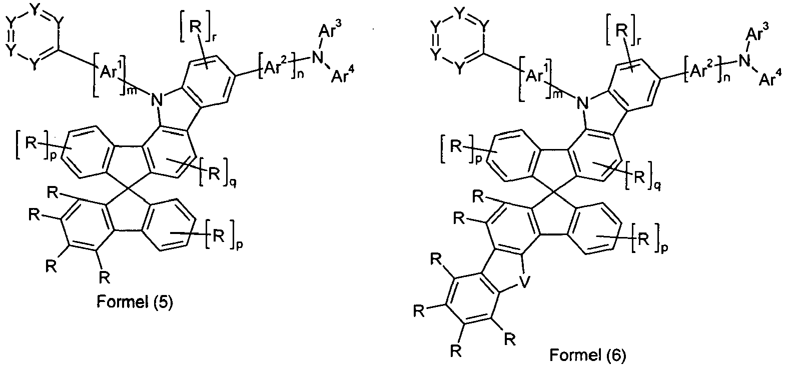 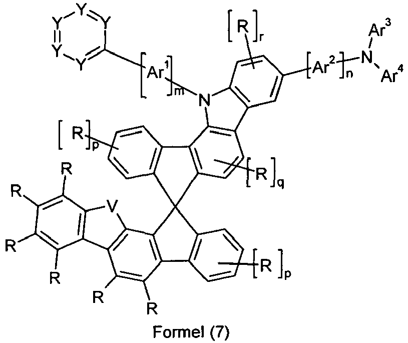 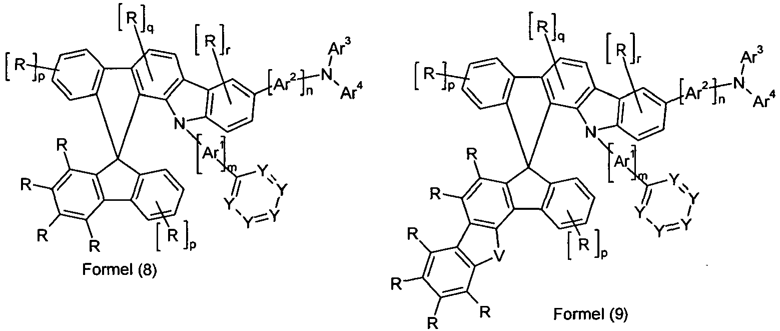 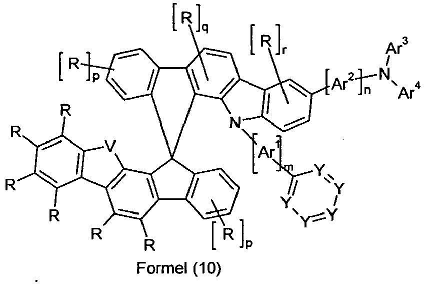 wobei die verwendeten Symbole und Indizes die in Anspruch 1 genannten Bedeutungen aufweisen.

4. Verbindung nach einem oder mehreren der Ansprüche 1 bis 3, **dadurch gekennzeichnet, dass** p bei jedem Auftreten gleich oder verschieden 0, 1 oder 2 ist und dass q gleich 0 oder 1 ist und dass r gleich 0 oder 1 ist.

5. Verbindung nach einem oder mehreren der Ansprüche 1 bis 4, ausgewählt aus den Verbindungen der Formeln (5a) bis (10a), 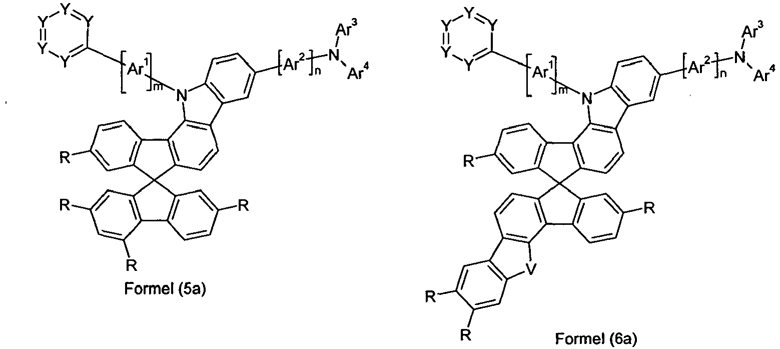 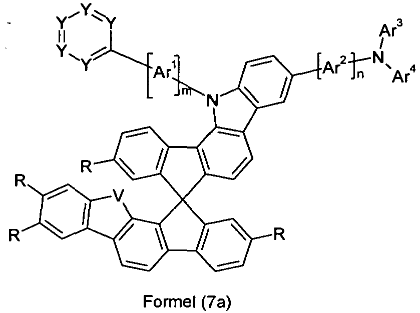 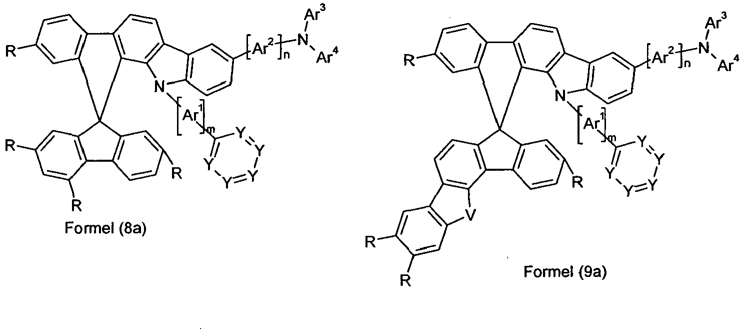 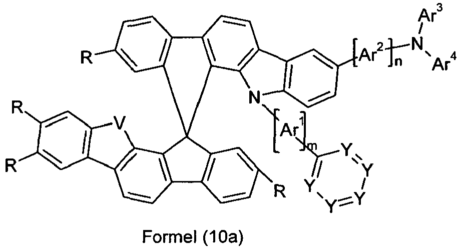 wobei die verwendeten Symbole und Indizes die in Anspruch 1 genannten Bedeutungen aufweisen.

6. Verbindung nach einem oder mehreren der Ansprüche 1 bis 5, ausgewählt aus den Verbindungen der Formeln (5b) bis (10b), 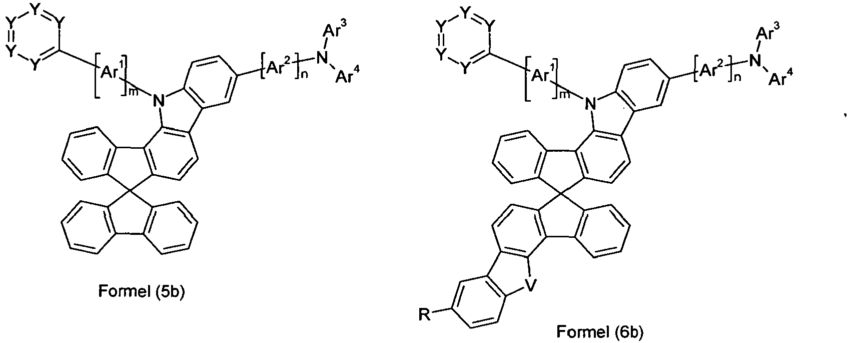 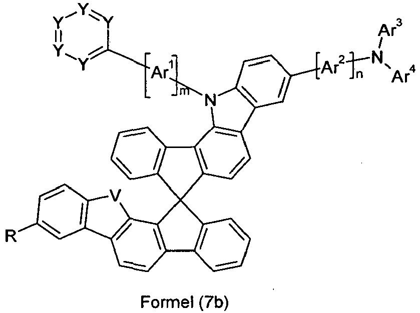 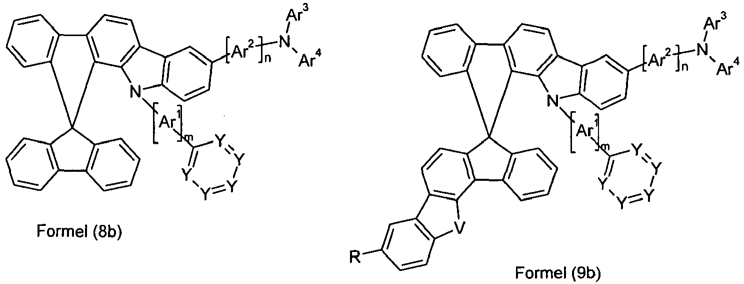 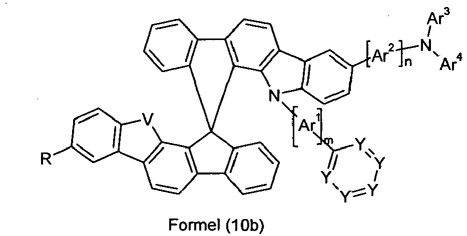 wobei die verwendeten Symbole und Indizes die in Anspruch 1 genannten Bedeutungen aufweisen.

7. Verbindung nach einem oder mehreren der Ansprüche 1 bis 6, **dadurch gekennzeichnet, dass** R gleich oder verschieden bei jedem Auftreten ausgewählt ist aus der Gruppe bestehend aus H, D, F, CN, N(Ar⁵)₂, C(=O)Ar⁵, P(=O)(Ar⁵)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R¹ substituiert sein kann.

8. Verbindung nach einem oder mehreren der Ansprüche 1 bis 7, **dadurch gekennzeichnet, dass** die Gruppe 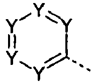 in Formel (1) bzw. Formel (2) ausgewählt ist aus den Gruppen der Formeln (Het-Ar-1) bis (Het-Ar-10), 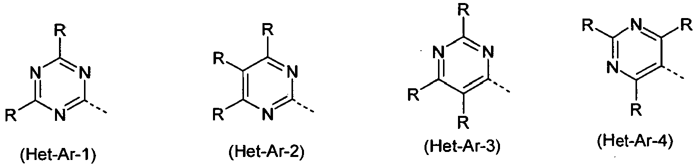 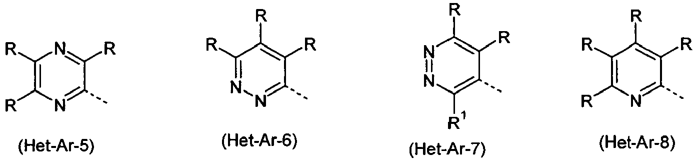 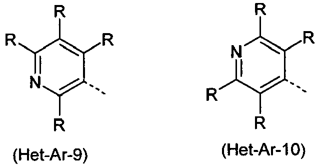 wobei die gestrichelte Bindung die Bindung an Ar¹ bzw. für m = 0 die Bindung an den Stickstoff darstellt und R gleich oder verschieden bei jedem Auftreten für H, D oder ein aromatisches oder heteroaromatisches Ringsystem mit 5 bis 24 aromatischen Ringatomen steht, welches durch einen oder mehrere Reste R¹ substituiert sein kann.

9. Verbindung nach einem oder mehreren der Ansprüche 1 bis 8, **dadurch gekennzeichnet, dass** n = 1 ist und Ar² und Ar³ miteinander durch eine Einfachbindung verbunden sind oder dass n = 0 oder 1 ist und Ar³ und Ar⁴ miteinander durch eine Einfachbindung verbunden sind oder dass n = 0 oder 1 ist und keine der Gruppen Ar², Ar³ bzw. Ar⁴ miteinander verbunden sind.

10. Verbindung nach einem oder mehreren der Ansprüche 1 bis 9, **dadurch gekennzeichnet, dass** Ar² ein aromatisches oder heteroaromatisches Ringsystem mit 6 bis 24 aromatischen Ringatomen ist, das mit einem oder mehreren Resten R substituiert sein kann, bevorzugt ausgewählt aus der Gruppe bestehend aus Phenylen oder Biphenyl, welche jeweils durch einen oder mehrere Reste R substituiert sein können, und dass Ar³ und Ar⁴ gleich oder verschieden bei jedem Auftreten ein aromatisches oder heteroaromatisches Ringsystem mit 6 bis 24 aromatischen Ringatomen darstellen, das jeweils mit einem oder mehreren Resten R substituiert sein kann, bevorzugt ausgewählt aus der Gruppe bestehend aus Phenyl, Biphenyl, Terphenyl, Quaterphenyl, Fluorenyl, Spirobifluorenyl, Naphthyl, Indolyl, Benzofuranyl, Benzothiophenyl, Carbazolyl, Dibenzofuranyl, Dibenzothiophenyl, Indenocarbazolyl, Indolocarbazolyl, Pyridinyl, Pyrimidinyl, Pyrazinyl, Pyridazinyl, Triazinyl, Phenanthrenyl, Triphenylenyl oder Kombinationen aus zwei, drei oder vier dieser Gruppen, welche jeweils mit einem oder mehreren Resten R substituiert sein können.

11. Verbindung nach einem oder mehreren der Ansprüche 1 bis 10, **dadurch gekennzeichnet, dass** die Gruppe -[Ar²]ₙ-N(Ar³)(Ar⁴) in Formel (1) bzw. Formel (2) ausgewählt ist aus den Gruppen der Formeln (CARB-1) bis (CARB-5), 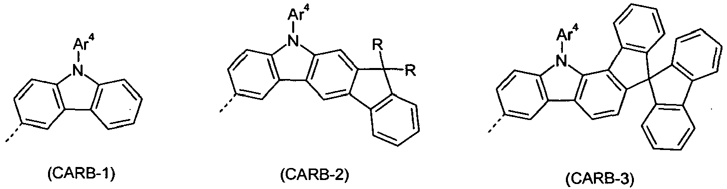 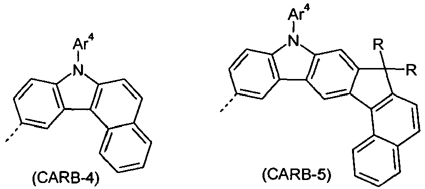 wobei Ar⁴ die in Anspruch 1 genannten Bedeutungen aufweist und die Gruppen durch einen oder mehrere Reste R substituiert sein können.

12. Formulierung, enthaltend mindestens eine Verbindung nach einem oder mehreren der Ansprüche 1 bis 11 und mindestens eine weitere Verbindung, insbesondere mindestens ein Lösemittel.

13. Verwendung einer Verbindung nach einem oder mehreren der Ansprüche 1 bis 11 oder einer Formulierung nach Anspruch 12 in einer elektronischen Vorrichtung.

14. Elektronische Vorrichtung enthaltend mindestens eine Verbindung nach einem oder mehreren der Ansprüche 1 bis 11.

15. Elektronische Vorrichtung nach Anspruch 14, **dadurch gekennzeichnet, dass** es sich um eine organische Elektrolumineszenzvorrichtung handelt, wobei die Verbindung nach einem oder mehreren der Ansprüche 1 bis 11 als Matrixmaterial für phosphoreszierende Emitter in einer emittierenden Schicht eingesetzt wird.

16. Elektronische Vorrichtung nach Anspruch 15, **dadurch gekennzeichnet, dass** die Verbindung nach einem oder mehreren der Ansprüche 1 bis 11 als Matrixmaterial für einen phosphoreszierenden Emitter in Kombination mit einem weiteren Matrixmaterial eingesetzt wird, wobei es sich bei dem weiteren Matrixmaterial bevorzugt um einen reinen Kohlenwasserstoff handelt.

## Claims

1. Compound, **characterised in that** the compound has a structure of the formula (1) or formula (2), 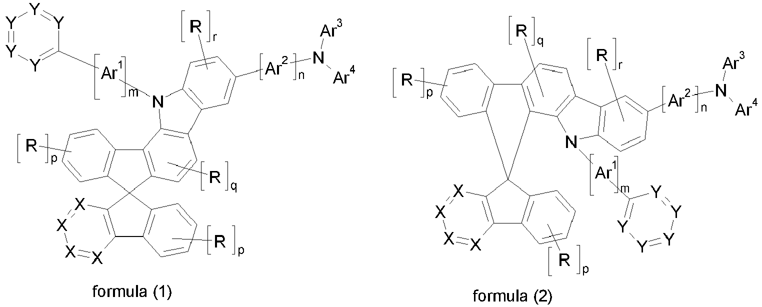 where the following applies to the symbols and indices used:
Y is on each occurrence, identically or differently, CR or N, with the proviso that at least one group Y stands for N;
X is on each occurrence, identically or differently, CR or N, where a maximum of two groups X stand for N; or two adjacent X stand for a group of the following formula (3) or (4) and the other groups X stand, identically or differently, for CR or N, 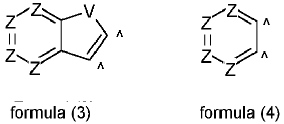 where ^ indicates the corresponding adjacent groups X in formula (1) or formula (2);
V is on each occurrence, identically or differently, NR, C(R)₂, O, S, BR, Si(R)₂ or C=O;
Z is on each occurrence, identically or differently, CR or N, where a maximum of two groups Z stand for N;
Ar¹ is an aromatic or heteroaromatic ring system having 5 to 40 aromatic ring atoms, which may be substituted by one or more radicals R;
Ar², Ar³, Ar⁴ are on each occurrence, identically or differently, an aromatic or heteroaromatic ring system having 5 to 40 aromatic ring atoms, which may be substituted by one or more radicals R; Ar² and Ar³ and/or Ar³ and Ar⁴ may also be connected to one another by a single bond or by a group selected from C(R¹)₂, C(R¹)₂-C(R¹)₂, NR¹, O or S;
R is selected on each occurrence, identically or differently, from the group consisting of H, D, F, Cl, Br, I, CN, NO₂, N(Ar⁵)₂, N(R¹)₂, C(=O)Ar⁵, C(=O)R¹, P(=O)(Ar⁵)₂, P(Ar⁵)₂, B(Ar⁵)₂, Si(Ar⁵)₃, Si(R¹)₃, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R¹, where one or more non-adjacent CH₂ groups may be replaced by R¹C=CR¹, C≡C, Si(R¹)₂, C=O, C=S, C=NR¹, P(=O)(R¹), SO, SO₂, NR¹, O, S or CONR¹ and where one or more H atoms may be replaced by D, F, Cl, Br, I, CN or NO₂, an aromatic or heteroaromatic ring system having 5 to 40 aromatic ring atoms, which may in each case be substituted by one or more radicals R¹, an aryloxy or heteroaryloxy group having 5 to 40 aromatic ring atoms, which may be substituted by one or more radicals R¹; two adjacent substituents R may optionally form a monocyclic or polycyclic, aliphatic, aromatic or heteroaromatic ring system, which may be substituted by one or more radicals R¹;
Ar⁵ is on each occurrence, identically or differently, an aromatic or heteroaromatic ring system having 5 to 30 aromatic ring atoms, which may be substituted by one or more non-aromatic radicals R¹; two radicals Ar⁵ which are bonded to the same N, P, B or Si atom may also be bridged to one another by a single bond or a bridge selected from N(R¹), C(R¹)₂, C(R¹)₂-C(R¹)₂, O or S;
R¹ is selected on each occurrence, identically or differently, from the group consisting of H, D, F, CN, an aliphatic hydrocarbon radical having 1 to 20 C atoms, or an aromatic or heteroaromatic ring system having 5 to 30 aromatic ring atoms, in which one or more H atoms may be replaced by D, F, CN or an alkyl group having 1 to 10 C atoms, where two or more adjacent substituents R¹ may form a mono- or polycyclic, aliphatic ring system with one another;
m is 0 or 1;
n is 0 or 1;
p is on each occurrence, identically or differently, 0, 1, 2, 3 or 4;
q is 0, 1 or 2;
r is 0, 1, 2 or 3;
the following compound is excluded from the invention:

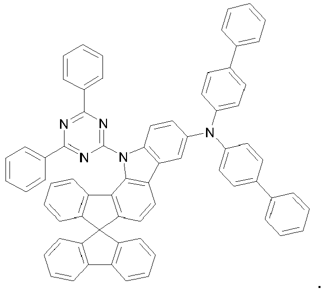

2. Compound according to Claim 1, **characterised in that** X stands, identically or differently on each occurrence, for CR or N, where a maximum of one group X per ring stands for N; or two adjacent groups X stand for a group of the formula (3), where Z stands, identically or differently on each occurrence, for CR and V stands for NR, C(R)₂, O or S, and the other X stand for CR.

3. Compound according to Claim 1 or 2, selected from the compounds of the formulae (5) to (10), 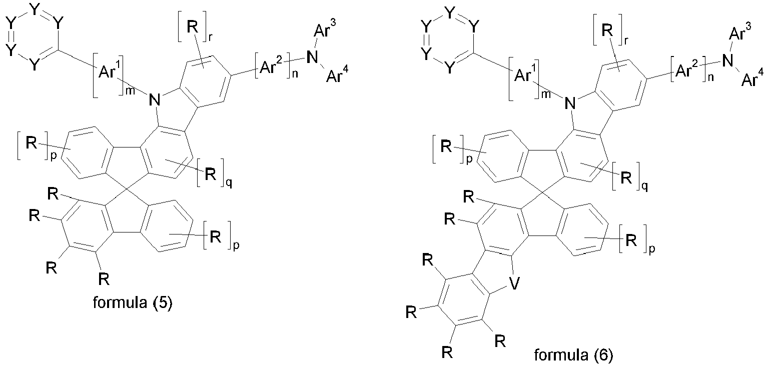 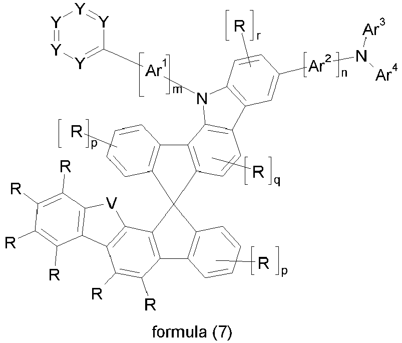 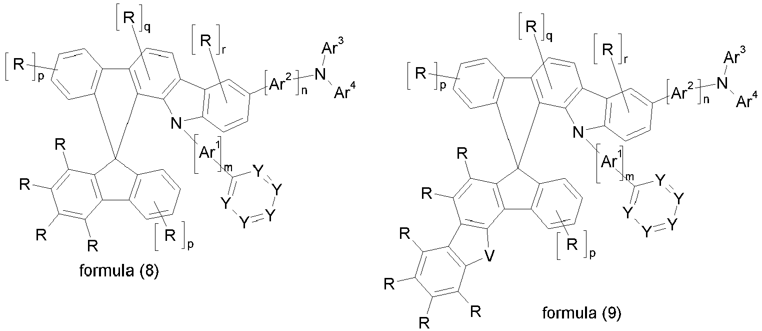 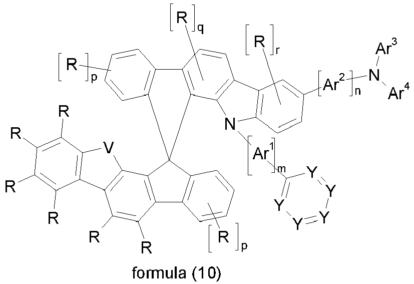 where the symbols and indices used have the meanings given in Claim 1.

4. Compound according to one or more of Claims 1 to 3, **characterised in that** p is on each occurrence, identically or differently, 0, 1 or 2 and **in that** q is equal to 0 or 1 and **in that** r is equal to 0 or 1.

5. Compound according to one or more of Claims 1 to 4, selected from the compounds of the formulae (5a) to (10a), 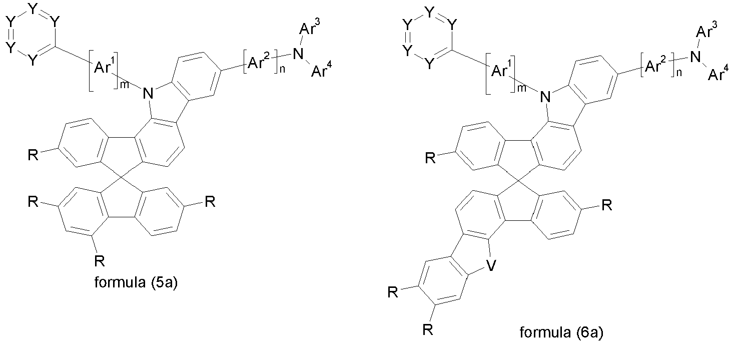 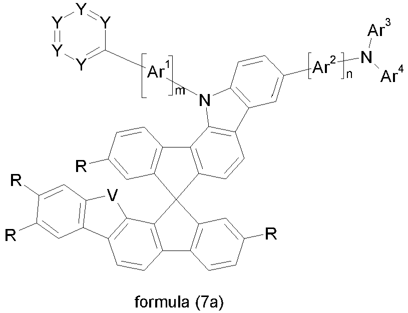 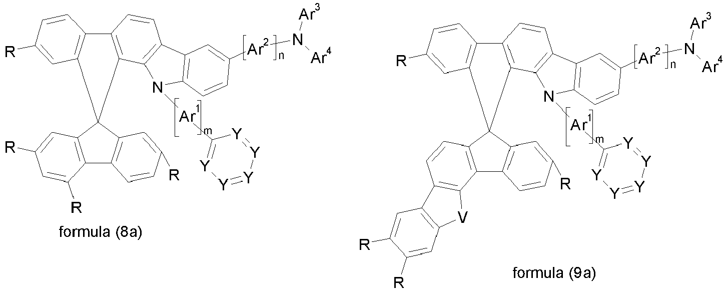 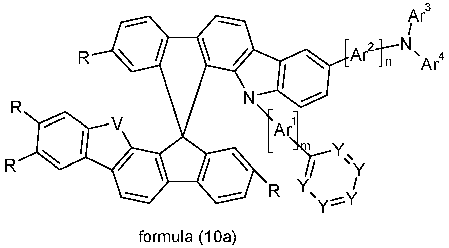 where the symbols and indices used have the meanings given in Claim 1.

6. Compound according to one or more of Claims 1 to 5, selected from the compounds of the formulae (5b) to (10b), 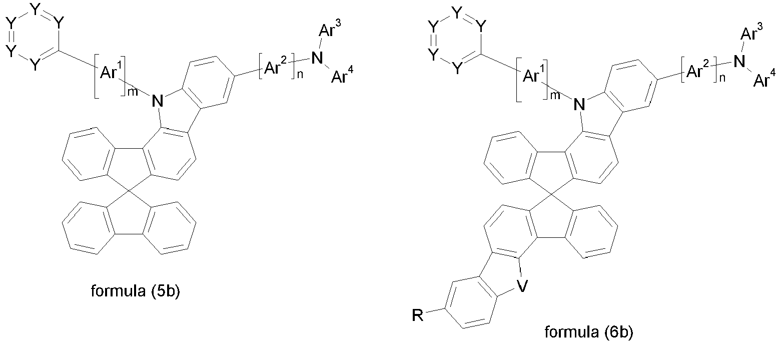 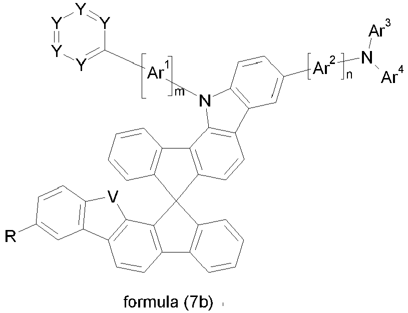 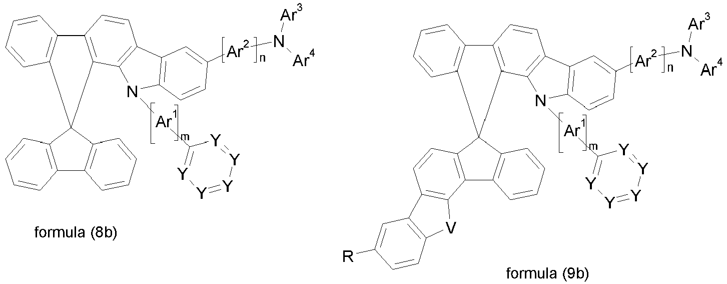 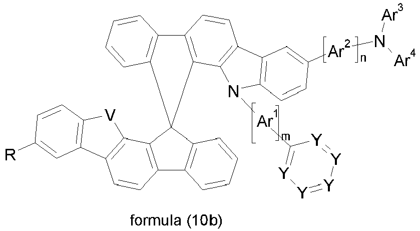 where the symbols and indices used have the meanings given in Claim 1.

7. Compound according to one or more of Claims 1 to 6, **characterised in that** R is selected, identically or differently on each occurrence, from the group consisting of H, D, F, CN, N(Ar⁵)₂, C(=O)Ar⁵, P(=O)(Ar⁵)₂, a straight-chain alkyl or alkoxy group having 1 to 10 C atoms or a branched or cyclic alkyl or alkoxy group having 3 to 10 C atoms or an alkenyl or alkynyl group having 2 to 10 C atoms, which may in each case be substituted by one or more radicals R¹, where one or more non-adjacent CH₂ groups may be replaced by O and where one or more H atoms may be replaced by D or F, or an aromatic or heteroaromatic ring system having 5 to 30 aromatic ring atoms, which may in each case be substituted by one or more radicals R¹.

8. Compound according to one or more of Claims 1 to 7, **characterised in that** the group 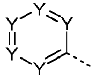 in formula (1) or formula (2) is selected from the groups of the formulae (Het-Ar-1) to (Het-Ar-10), 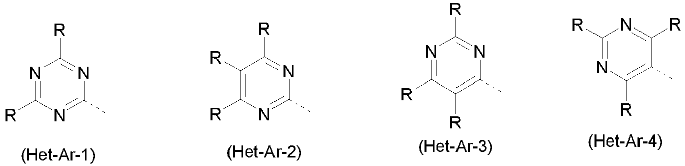 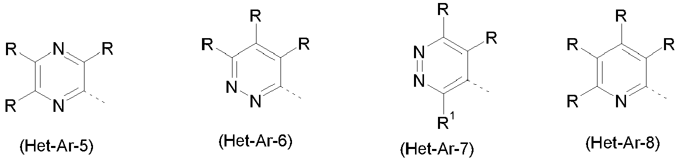 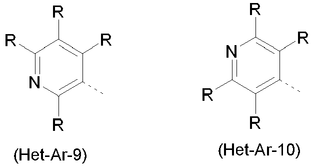 where the dashed bond represents the bond to Ar¹ or, for m = 0, the bond to the nitrogen and R stands, identically or differently on each occurrence, for H, D or an aromatic or heteroaromatic ring system having 5 to 24 aromatic ring atoms, which may be substituted by one or more radicals R¹.

9. Compound according to one or more of Claims 1 to 8, **characterised in that** n = 1 and Ar² and Ar³ are connected to one another by a single bond or **in that** n = 0 or 1 and Ar³ and Ar⁴ are connected to one another by a single bond or **in that** n = 0 or 1 and none of the groups Ar², Ar³ and Ar⁴ are connected to one another.

10. Compound according to one or more of Claims 1 to 9, **characterised in that** Ar² is an aromatic or heteroaromatic ring system having 6 to 24 aromatic ring atoms, which may be substituted by one or more radicals R, preferably selected from the group consisting of phenylene or biphenyl, which may in each case be substituted by one or more radicals R, and **in that** Ar³ and Ar⁴ represent, identically or differently on each occurrence, an aromatic or heteroaromatic ring system having 6 to 24 aromatic ring atoms, which may in each case be substituted by one or more radicals R, preferably selected from the group consisting of phenyl, biphenyl, terphenyl, quaterphenyl, fluorenyl, spirobifluorenyl, naphthyl, indolyl, benzofuranyl, benzothiophenyl, carbazolyl, dibenzofuranyl, dibenzothiophenyl, indenocarbazolyl, indolocarbazolyl, pyridinyl, pyrimidinyl, pyrazinyl, pyridazinyl, triazinyl, phenanthrenyl, triphenylenyl or combinations of two, three or four of these groups, which may in each case be substituted by one or more radicals R.

11. Compound according to one or more of Claims 1 to 10, **characterised in that** the group -[Ar²]ₙ-N(Ar³)(Ar⁴) in formula (1) or formula (2) is selected from the groups of the formulae (CARB-1) to (CARB-5), 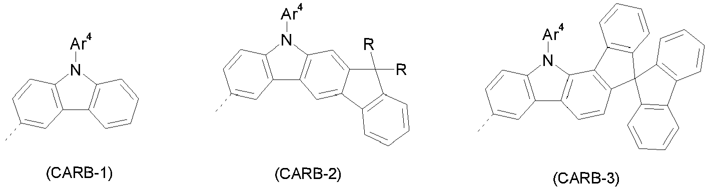 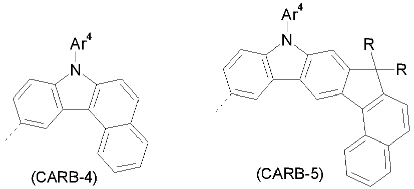 where Ar⁴ has the meanings given in Claim 1 and the groups may be substituted by one or more radicals R.

12. Formulation comprising at least one compound according to one or more of Claims 1 to 11 and at least one further compound, in particular at least one solvent.

13. Use of a compound according to one or more of Claims 1 to 11 or a formulation according to Claim 12 in an electronic device.

14. Electronic device comprising at least one compound according to one or more of Claims 1 to 11.

15. Electronic device according to Claim 14, **characterised in that** it is an organic electroluminescent device, where the compound according to one or more of Claims 1 to 11 is employed as matrix material for phosphorescent emitters in an emitting layer.

16. Electronic device according to Claim 15, **characterised in that** the compound according to one or more of Claims 1 to 11 is employed as matrix material for a phosphorescent emitter in combination with a further matrix material, where the further matrix material is preferably a pure hydrocarbon.

## Revendications

1. Composé, **caractérisé en ce que** le composé présente une structure de la formule (1) ou de la formule (2), 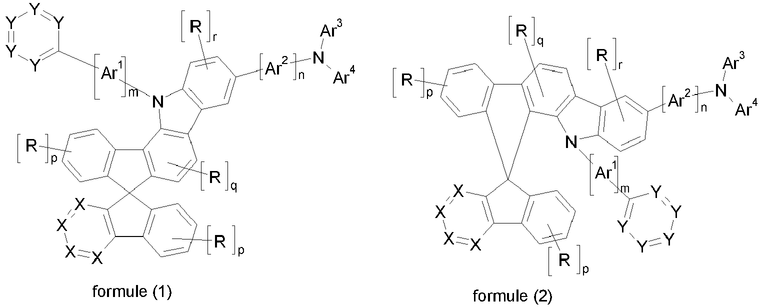 dans lesquelles ce qui suit s'applique aux symboles et aux indices qui sont utilisés :
Y est pour chaque occurrence, de manière identique ou différente, CR ou N, étant entendu qu'au moins un groupe Y représente N ;
X est pour chaque occurrence, de manière identique ou différente, CR ou N, où un maximum de deux groupes X représentent N ; ou deux groupes X adjacents représentent un groupe de la formule qui suit (3) ou (4) et les autres groupes X représentent, de manière identique ou différente CR ou N, 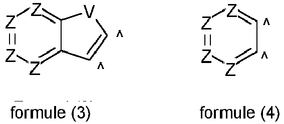 dans lesquelles ^ indique les groupes X adjacents correspondants dans la formule (1) ou dans la formule (2) ;
V est pour chaque occurrence, de manière identique ou différente, NR, C(R)₂, O, S, BR, Si(R)₂ ou C=O ;
Z est pour chaque occurrence, de manière identique ou différente, CR ou N, où un maximum de deux groupes Z représentent N ;
Ar¹ est un système de cycle aromatique ou hétéroaromatique qui comporte de 5 à 40 atomes de cycle aromatique, lequel peut être substitué par un radical ou par plusieurs radicaux R ;
Ar², Ar³, Ar⁴ sont pour chaque occurrence, de manière identique ou différente, un système de cycle aromatique ou hétéroaromatique qui comporte de 5 à 40 atomes de cycle aromatique, lequel peut être substitué par un radical ou par plusieurs radicaux R ; Ar² et Ar³ et/ou Ar³ et Ar⁴ peuvent également être connectés l'un à l'autre ou les uns aux autres par une liaison simple ou par un groupe qui est sélectionné parmi C(R¹)₂, C(R¹)₂-C(R¹)₂, NR¹, O ou S ;
R est sélectionné pour chaque occurrence, de manière identique ou différente, parmi le groupe qui est constitué par H, D, F, Cl, Br, I, CN, NO₂, N(Ar⁵)₂, N(R¹)₂, C(=O)Ar⁵, C(=O)R¹, P(=O)(Ar⁵)₂, P(Ar⁵)₂, B(Ar⁵)₂, Si(Ar⁵)₃, Si(R¹)₃, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, lequel peut dans chaque cas être substitué par un radical ou par plusieurs radicaux R¹, où un ou plusieurs groupe(s) CH₂ non adjacents peut/peuvent être remplacé(s) par R¹C=CR¹, C≡C, Si(R¹)₂, C=O, C=S, C=NR¹, P(=O)(R¹), SO, SO₂, NR¹, O, S ou CONR¹ et où un ou plusieurs atome(s) de H peut/ peuvent être remplacé(s) par D, F, Cl, Br, I, CN ou NO₂, un système de cycle aromatique ou hétéroaromatique qui comporte de 5 à 40 atomes de cycle aromatique, lequel peut dans chaque cas être substitué par un radical ou par plusieurs radicaux R¹, un groupe aryloxy ou hétéroaryloxy qui comporte de 5 à 40 atomes de cycle aromatique, lequel peut être substitué par un radical ou par plusieurs radicaux R¹ ; deux substituants R adjacents peuvent en option former un système de cycle aliphatique, aromatique ou hétéroaromatique monocyclique ou polycyclique, lequel peut être substitué par un radical ou par plusieurs radicaux R¹ ;
Ar⁵ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹ non aromatiques ; deux radicaux Ar⁵ qui sont liés au même atome de N, P, B ou Si peuvent également être pontés l'un à l'autre par une liaison simple ou par un pont qui est sélectionné parmi N(R¹), C(R¹)₂, C(R¹)₂-C(R¹)₂, O ou S ;
R¹ est sélectionné pour chaque occurrence, de manière identique ou différente, parmi le groupe qui est constitué par H, D, F, CN, un radical hydrocarbone aliphatique qui comporte de 1 à 20 atome(s) de C, ou un système de cycle aromatique ou hétéroaromatique qui comporte de 5 à 30 atomes de cycle aromatique, dans lequel un ou plusieurs atome(s) de H peut/peuvent être remplacé(s) par D, F, CN ou un groupe alkyle qui comporte de 1 à 10 atome(s) de C, où deux substituants R¹ adjacents ou plus peuvent former un système de cycle aliphatique mono- ou polycyclique l'un avec l'autre ou les uns avec les autres ;
m est 0 ou 1 ;
n est 0 ou 1 ;
p est pour chaque occurrence, de manière identique ou différente, 0, 1, 2, 3 ou 4 ;
q est 0, 1 ou 2 ;
r est 0, 1, 2 ou 3 ;
le composé qui suit est exclu de l'invention :

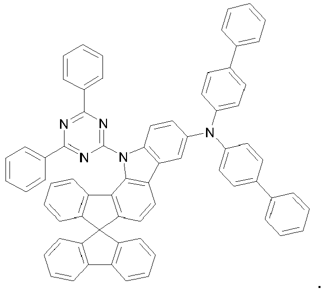

2. Composé selon la revendication 1, **caractérisé en ce que** X représente, de manière identique ou différente pour chaque occurrence, CR ou N, où un maximum d'un groupe X par cycle représente N ; ou deux groupes X adjacents représentent un groupe de la formule (3), où Z représente, de manière identique ou différente pour chaque occurrence, CR et V représente NR, C(R)₂, O ou S, et l'autre groupe X représente CR.

3. Composé selon la revendication 1 ou 2, sélectionné parmi les composés des formules (5) à (10), 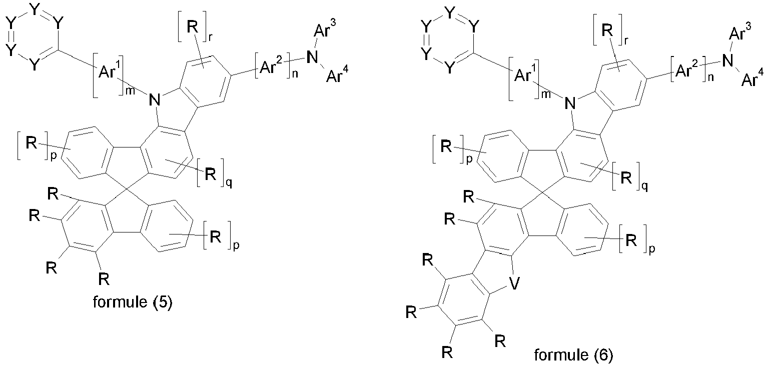 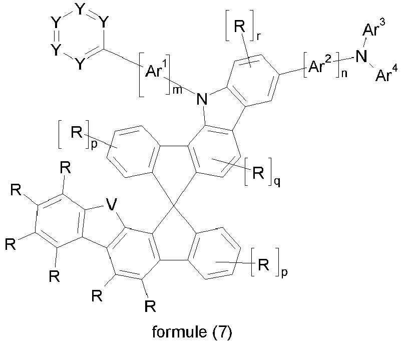 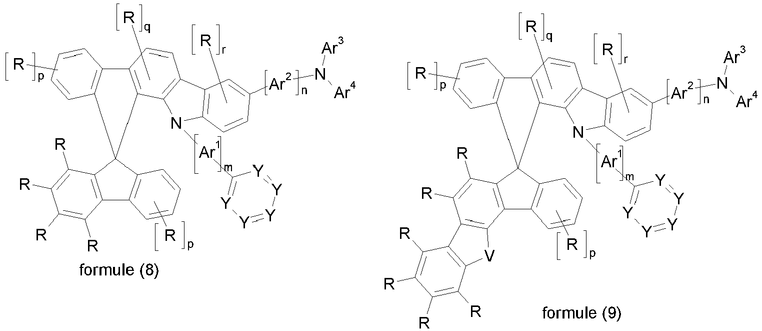 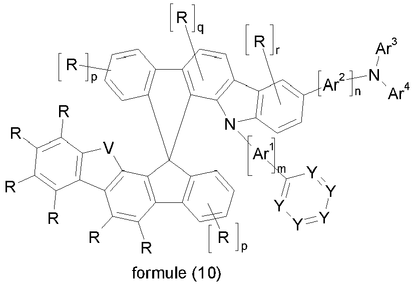 dans lesquelles les symboles et les indices qui sont utilisés présentent les significations qui ont été données selon la revendication 1.

4. Composé selon une ou plusieurs des revendications 1 à 3, **caractérisé en ce que** p est pour chaque occurrence, de manière identique ou différente, 0, 1 ou 2 et **en ce que** q est égal à 0 ou 1 et **en ce que** r est égal à 0 ou 1.

5. Composé selon une ou plusieurs des revendications 1 à 4, sélectionné parmi les composés des formules (5a) à (10a), 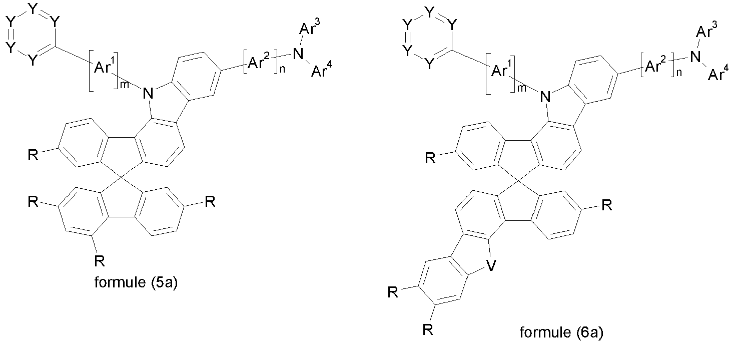 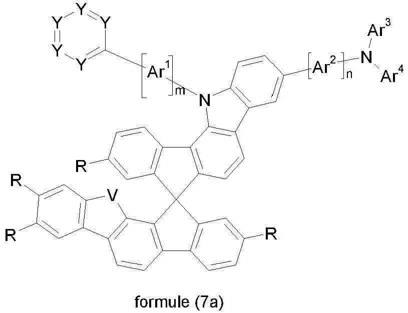 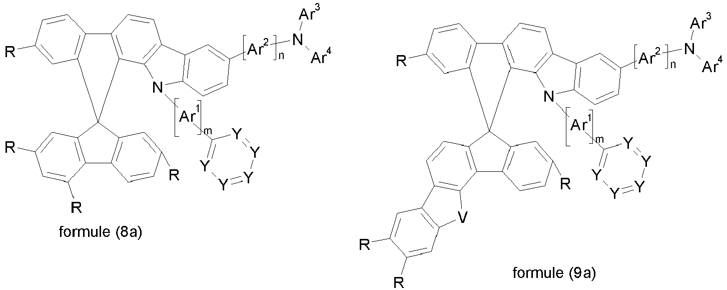 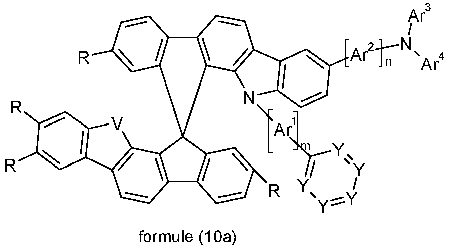 dans lesquelles les symboles et les indices qui sont utilisés présentent les significations qui ont été données selon la revendication 1.

6. Composé selon une ou plusieurs des revendications 1 à 5, sélectionné parmi les composés des formules (5b) à (10b), 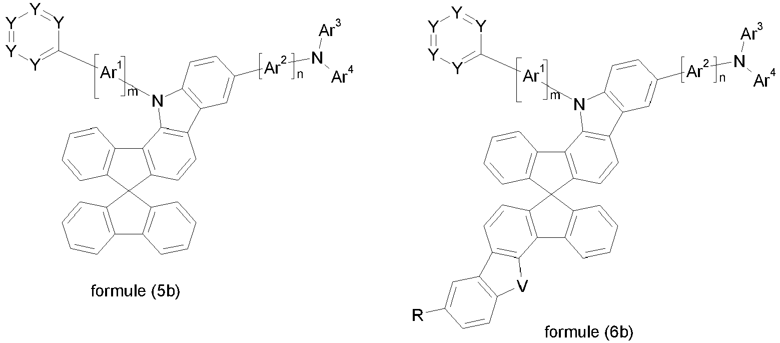 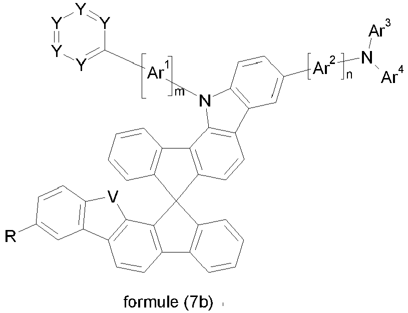 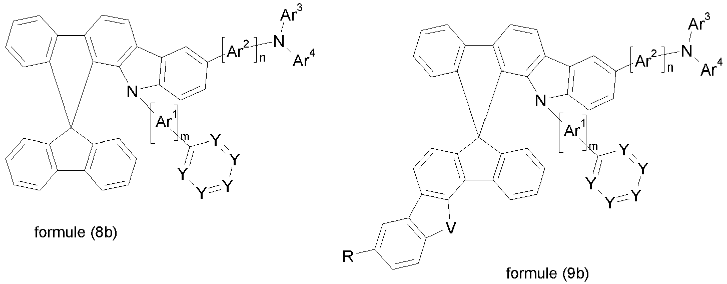 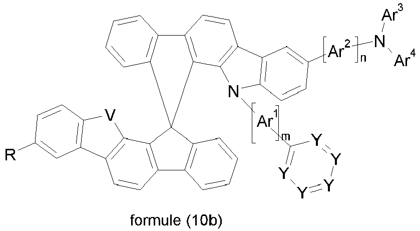 dans lesquelles les symboles et les indices qui sont utilisés présentent les significations qui ont été données selon la revendication 1.

7. Composé selon une ou plusieurs des revendications 1 à 6, **caractérisé en ce que** R est sélectionné, de manière identique ou différente pour chaque occurrence, parmi le groupe qui est constitué par H, D, F, CN, N(Ar⁵)₂, C(=O)Ar⁵, P(=O)(Ar⁵)₂, un groupe alkyle ou alcoxy en chaîne droite qui comporte de 1 à 10 atome(s) de C ou un groupe alkyle ou alcoxy ramifié ou cyclique qui comporte de 3 à 10 atomes de C ou un groupe alkényle ou alkynyle qui comporte de 2 à 10 atomes de C, lequel peut dans chaque cas être substitué par un radical ou par plusieurs radicaux R¹, où un ou plusieurs groupe(s) CH₂ non adjacents peut/peuvent être remplacé(s) par O et où un ou plusieurs atome(s) de H peut/peuvent être remplacé(s) par D ou par F, ou un système de cycle aromatique ou hétéroaromatique qui comporte de 5 à 30 atomes de cycle aromatique, lequel peut dans chaque cas être substitué par un radical ou par plusieurs radicaux R¹.

8. Composé selon une ou plusieurs des revendications 1 à 7, **caractérisé en ce que** le groupe 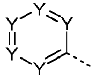 dans la formule (1) ou la formule (2) est sélectionné parmi les groupes des formules (Het-Ar-1) à (Het-Ar-10), 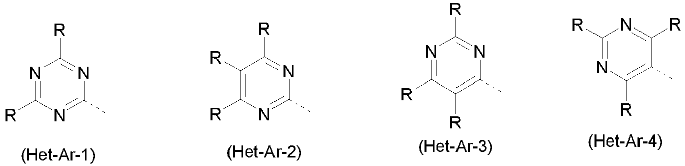 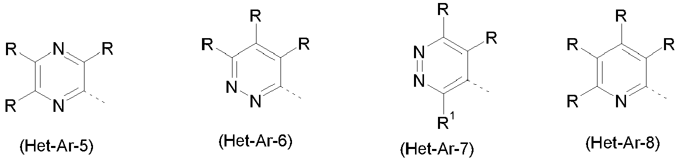 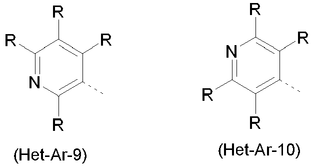 dans lesquelles les lignes en pointillés représentent la liaison sur Ar¹ ou, pour m = 0, la liaison sur l'azote et R représente, de manière identique ou différente pour chaque occurrence, H, D, ou un système de cycle aromatique ou hétéroaromatique qui comporte de 5 à 24 atomes de cycle aromatique, lequel peut être substitué par un radical ou par plusieurs radicaux R¹.

9. Composé selon une ou plusieurs des revendications 1 à 8, **caractérisé en ce que** n = 1 et Ar² et Ar³ sont connectés l'un à l'autre par une liaison simple ou **en ce que** n = 0 ou 1 et Ar³ et Ar⁴ sont connectés l'un à l'autre par une liaison simple ou **en ce que** n = 0 ou 1 et aucun des groupes Ar², Ar³ et Ar⁴ n'est connecté à un autre groupe d'entre eux.

10. Composé selon une ou plusieurs des revendications 1 à 9, **caractérisé en ce que** Ar² est un système de cycle aromatique ou hétéroaromatique qui comporte de 6 à 24 atomes de cycle aromatique, lequel peut être substitué par un radical ou par plusieurs radicaux R, de préférence sélectionné parmi le groupe qui est constitué par phénylène ou biphényle, lequel peut dans chaque cas être substitué par un radical ou par plusieurs radicaux R, et **en ce que** Ar³ et Ar⁴ représentent, de manière identique ou différente pour chaque occurrence, un système de cycle aromatique ou hétéroaromatique qui comporte de 6 à 24 atomes de cycle aromatique, lequel peut dans chaque cas être substitué par un radical ou par plusieurs radicaux R, de préférence sélectionné parmi le groupe qui est constitué par phényle, biphényle, terphényle, quaterphényle, fluorényle, spirobifluorényle, naphtyle, indolyle, benzofuranyle, benzothiophényle, carbazolyle, dibenzofuranyle, dibenzothiophényle, indénocarbazolyle, indolocarbazolyle, pyridinyle, pyrimidinyle, pyrazinyle, pyridazinyle, triazinyle, phénanthrényle, triphénylényle ou des combinaisons de deux, trois ou quatre de ces groupes, lequel peut dans chaque cas être substitué par un radical ou par plusieurs radicaux R.

11. Composé selon une ou plusieurs des revendications 1 à 10, **caractérisé en ce que** le groupe -[Ar²]ₙ-N(Ar³)(Ar⁴) dans la formule (1) ou la formule (2) est sélectionné parmi les groupes des formules (CARB-1) à (CARB-5), 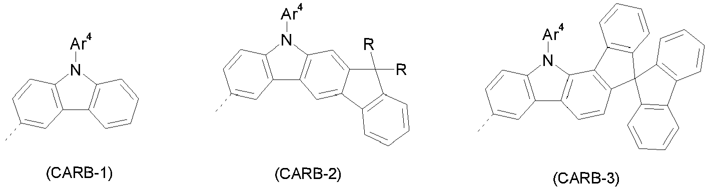 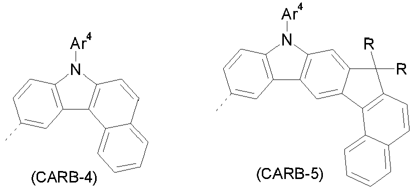 dans lesquelles Ar⁴ présente les significations qui ont été données selon la revendication 1 et les groupes peuvent être substitués par un radical ou par plusieurs radicaux R.

12. Formulation comprenant au moins un composé selon une ou plusieurs des revendications 1 à 11 et au moins un autre composé, en particulier au moins un solvant.

13. Utilisation d'un composé selon une ou plusieurs des revendications 1 à 11 ou d'une formulation selon la revendication 12 dans un dispositif électronique.

14. Dispositif électronique comprenant au moins un composé selon une ou plusieurs des revendications 1 à 11.

15. Dispositif électronique selon la revendication 14, **caractérisé en ce qu'**il s'agit d'un dispositif électroluminescent organique, dans lequel le composé selon une ou plusieurs des revendications 1 à 11 est utilisé en tant que matériau de matrice pour des émetteurs phosphorescents dans une couche d'émission.

16. Dispositif électronique selon la revendication 15, **caractérisé en ce que** le composé selon une ou plusieurs des revendications 1 à 11 est utilisé en tant que matériau de matrice pour un émetteur phosphorescent en combinaison avec un autre matériau de matrice, dans lequel l'autre matériau de matrice est de préférence un hydrocarbone pur.
